# EUROPEAN PATENT APPLICATION

(11) **EP 4 667 019 A1**
(43) Date of publication of application: **24.12.2025**
(21) Application number: 23922166.6
(22) Date of filing: 19.04.2023
(51) Int. Cl.: A61K 51/04, C07D 257/02, C07D 403/12, A61K 47/65, A61P 35/00

(54) **PSMA-TARGETED RADIOPHARMACEUTICAL, AND SYNTHESIS AND USE THEREOF**

(30) Priority: 16.02.2023 CN 202310126134
(71) Applicant: Norroy Bioscience Co., Ltd., Jiangsu 214000 (CN)
(72) Inventor: YU, Shanyou, Wuxi, Jiangsu 214000 (CN); YAN, Chenglong, Wuxi, Jiangsu 214000 (CN); FANG, Peng, Wuxi, Jiangsu 214000 (CN); ENG, Wai-si, Wuxi, Jiangsu 214000 (CN); YANG, Si, Wuxi, Jiangsu 214000 (CN); YU, Defeng, Wuxi, Jiangsu 214000 (CN)
(74) Representative: Rigamonti, Dorotea
(86) International application number: PCT/CN2023/089311
(87) International publication number: WO 2024/169037

(57) **Abstract**

Provided are a PSMA-targeted radiopharmaceutical, and the synthesis and use thereof. Specifically, provided is a compound or a pharmaceutically acceptable salt, an ester or a solvate thereof and the structure thereof is as shown in formula (I). The compound can be used for diagnosing and/or treating one or more tumors, cancers, or cells expressing PSMA.

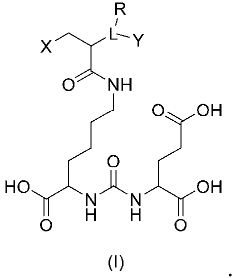

## Description

### PRIORITY INFORMATION

This application claims priority to and benefits of Patent Application No. 202310126134.7, filed with China National Intellectual Property Administration on February, 16, 2023, and the entire disclosure of which is incorporated herein by reference.

### FIELD

The present disclosure relates to the field of diagnosis, and particularly, to a PSMA-targeted radiopharmaceutical, and synthesis and use thereof.

### BACKGROUND

Prostate cancer is a common malignant tumor of a male genitourinary system and the second most common male tumor globally. In recent years, the incidence and detection rates of prostate cancer have been increasing year by year, and the mortality rate due to the disease has also been rising year by year. Currently, the tumors of most newly diagnosed patients have already developed local progression or distant metastasis, thus losing an opportunity for radical treatment. Therefore, how to treat patients with advanced-stage prostate cancer is one of the key issues in the current research field of prostate cancer treatment.

Prostate-specific membrane antigen (PSMA) is a unique type II transmembrane glycoprotein on a cell surface. PSMA has a binding site in its extracellular segment and has significantly enhanced cellular internalization activity after binding to relevant ligands. PSMA is positively expressed in almost all prostate cancers, and its expression level increases with the progression of tumor stage and grade. In the advanced-stage of prostate cancer and castration-resistant prostate cancer (CRPC), the expression of PSMA is significantly increased. This unique expression and internalization activity of PSMA after ligand binding make PSMA an important and ideal specific marker for prostate cancer, serving as a reliable extracellular target for reliable targeted molecular imaging and precision therapy. Research and development of a prostate cancer-targeted small molecule drug conjugate using PSMA as a target to treat metastatic CRPC has sufficient theoretical feasibility and practical application prospects.

While research related to a tumor-targeted therapy is progressing rapidly, researchers have further developed and refined it, proposing a new tumor treatment concept, theranostics of tumor. Theranostics combines diagnosis and treatment by integrating a diagnostic reagent and a therapeutic reagent within one system to achieve effects of imaging diagnosis and treatment simultaneously. This treatment strategy plays an important role in the field of personalized drug administration. It can not only effectively improve bioavailability and targeting of drugs, but also enhance anti-tumor activity and reduce its toxicity. Meanwhile, a drug delivery process in the body can be visualized with assistance of the imaging diagnosis, making it easier to understand the drug distribution in the body. Further, it is also possible to collect information on a disease state before and after the treatment and guide further medication, so as to realize real-time monitoring of the tumor treatment effect and the personalized drug administration. The tumor-targeted theranostics has become an important emerging branch in the field of biomedical research. This new technology, which integrates the imaging diagnosis and the targeted therapy and is constructed with multifunctional diagnostic and therapeutic agents, is expected to play an important role in diagnosis and treatment of human tumors or other major diseases in the future. It has promising application prospects and represents a hotspot and frontier area in current international biomedical research.

### SUMMARY

**In** a first aspect, the present disclosure provides a compound, or a pharmaceutically acceptable salt, ester, or solvate thereof. The compound has a structure represented by Formula (I): 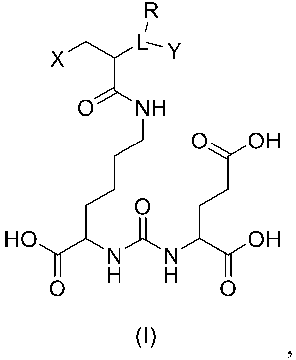 wherein: L is selected from a polyethylene glycol chain, a hydrophilic amino acid chain, or a carbon chain; X is selected from a six- to twelve-membered aryl or a five- to twelve-membered heteroaryl, wherein the six- to twelve-membered aryl or the five- to twelve-membered heteroaryl is optionally substituted with one, two, or three substituents selected from OH, halogen, or C₁₋₆ alkyl; and R and Y are respectively and independently selected from H, an optionally substituted amino acid, or a chelating agent, and at least one of R and Y is the chelating agent.

In some embodiments of the present disclosure, the above-mentioned polyethylene glycol chain is a chain formed by one or more polyethylene glycol units (-O-(CH₂)₂-O-), for example, a chain formed by 1, 2, 3, 4, 5, 6, 7, or 8 polyethylene glycol units (-O-(CH₂)₂-O-). The remaining variables are as defined in the present disclosure.

In some embodiments of the present disclosure, the above-mentioned hydrophilic amino acid chain is a chain formed by condensation of one or more same or different hydrophilic amino acids, for example, a chain formed by condensation of 1, 2, 3, 4, 5, 6, 7, or 8 hydrophilic amino acids. The term "hydrophilic amino acid" refers to a general term for amino acids with high hydrophilic side chains, for example, the hydrophilic amino acid includes threonine (Thr), serine (Ser), cysteine (Cys), asparagine (Asn), glutamine (Gln), tyrosine (Tyr), lysine (Lys), arginine (Arg), histidine (His), aspartic acid (Asp) or glutamic acid (Glu). The remaining variables are as defined in the present disclosure.

In some embodiments of the present disclosure, the above-mentioned carbon chain refers to a carbon chain formed by one or more substituted or unsubstituted linear alkyl or branched alkyl, for example, a carbon chain formed by a substituted or unsubstituted linear alkyl or branched alkyl composed of 1, 2, 3, 4, 5, 6, 7, or 8 carbon atoms. The remaining variables are as defined in the present disclosure.

In some embodiments of the present disclosure, L is selected from 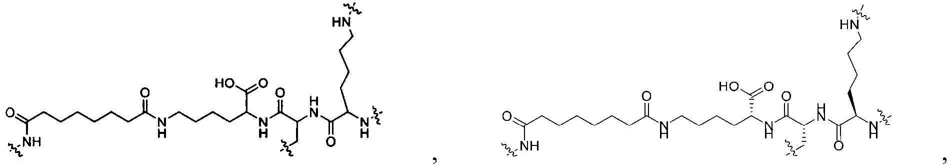 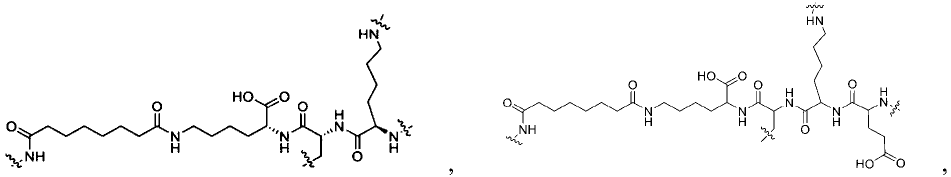 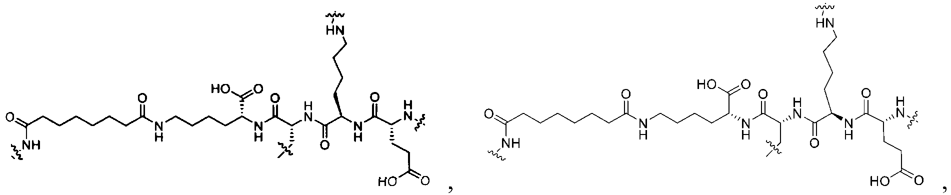 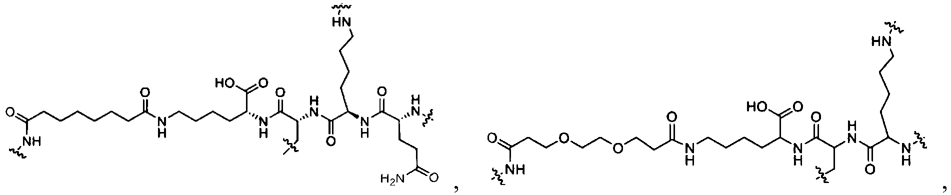 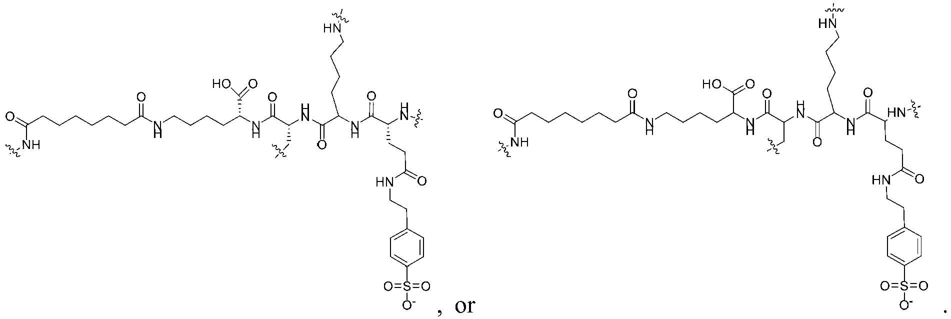 The remaining variables are as defined in the present disclosure.

In some embodiments of the present disclosure, R and Y are respectively and independently selected from H, 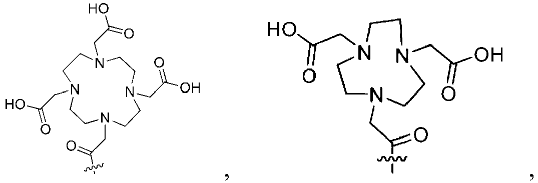 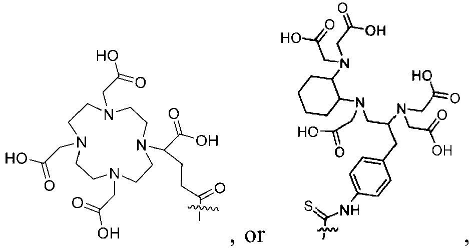 R and Y are not both H, and at least one of R and Y is the chelating agent.

In another aspect of the present disclosure, the present disclosure provides a compound, or a pharmaceutically acceptable salt, ester or solvate thereof. According to embodiments of the present disclosure, the compound has a structure represented by Formula (II): 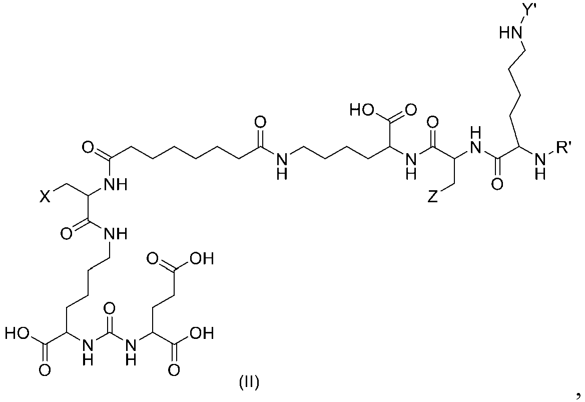 wherein: X is selected from a six- to twelve-membered aryl or a five- to twelve-membered heteroaryl, wherein the six- to twelve-membered aryl or five- to twelve-membered heteroaryl is optionally substituted with one, two, or three substituents selected from OH, halogen, or C₁₋₆ alkyl; Z is selected from 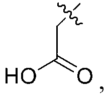 -COOH, -OH, C₁₋₆ alkyl, and six- to twelve-membered aryl, and the C₁₋₆ alkyl or the six- to twelve-membered aryl is optionally substituted with one, two, or three OH, halogen, or C₁₋₆ alkyl; and R' and Y' are each independently selected from H, an optionally substituted amino acid, or a chelating agent, and at least one of R' and Y' is the chelating agent.

In some embodiments of the present disclosure, R' and Y' are each independently selected from H, 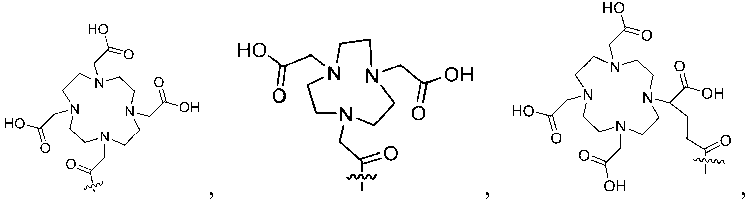 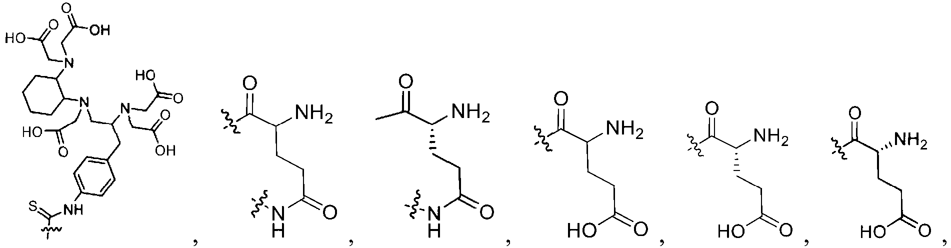 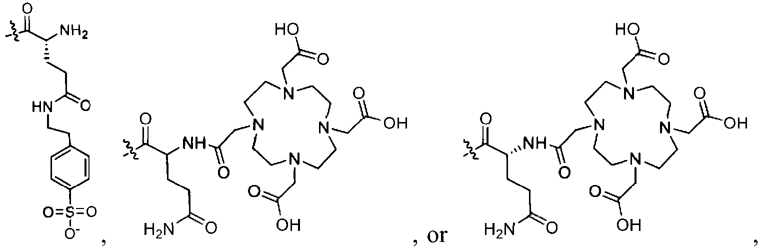 R' and Y' are not both H, and at least one of R' and Y' is the chelating agent.

In some embodiments of the present disclosure, when R' and Y' are independently an optionally substituted amino acid or a chelating group, the substituent is 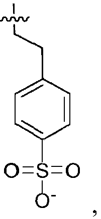 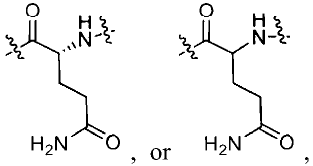 and the remaining variables are as defined in the present disclosure.

In some embodiments of the present disclosure, X is selected from 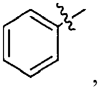 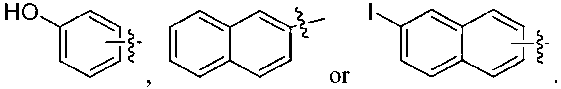 The remaining variables are as defined in the present disclosure.

In some embodiments of the present disclosure, Z is selected from 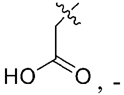 COOH, -OH, -CH(CH₃)OH, 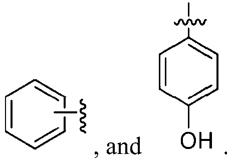 The remaining variables are as defined in the present disclosure.

In some embodiments of the present disclosure, the chelating agent is selected from 1,4,7,10-tetraazacyclododecane-N,N',N",N‴,-tetraacetic acid, 1,4,7-triazacyclononane-1,4,7-triacetic acid, 2-(4,7-bis(carboxymethyl)-1,4,7-triazanonan-1-yl)pentanedioic acid, 2-(4,7,10-tris(carboxymethyl)-1,4,7,10-tetraazacyclododecan-1-yl)pentanedioic acid, 1,4,7-triazacyclononane phosphinic acid, 1,4,7-triazacyclononane-1-[methyl(2-carboxyethyl)phosphinic acid]-4,7-bis[methyl(2-carboxymethyl)phosphinic acid], N'-{5-[acetyl(hydroxy)amino]pentyl}-N-[5-({4-[5-aminopentyl)(hydroxy)amino]-4-oxobutanoyl}amino)pentyl]-N-hydroxysuccinamide, diethylenetriaminepentaacetic acid, trans-cyclohexyl-diethylenetriaminepentaacetic acid, p-isothiocyanatobenzyl-diethylenetriaminepentaacetic acid, 1-(isothiocyanatobenzyl)-3-methyl-diethylenetriaminepentaacetic acid, 1-(isothiocyanatobenzyl)-4-methyl-diethylenetriaminepentaacetic acid, 1-(2)-methyl-4-isothiocyanatobenzyl-diethylenetriaminepentaacetic acid, 1-oxa-4,7,10-triazacyclododecane-4,7,10-triacetic acid, 6-hydrazinonicotinic acid succinimidyl ester hydrochloride, mercaptoacetyltriglycine, or [(R)-2-amino-3-(4-isothiocyanatophenyl)propyl]-trans-(S,S)-cyclohexane-1,2-diaminopentaacetic acid. The remaining variables are as defined in the present disclosure.

In yet another aspect of the present disclosure, the present disclosure also provides a complex. According to the embodiments of the present disclosure, the complex is formed by complexing the compound or the pharmaceutically acceptable salt, ester or solvate thereof described above with a radionuclide A' or a non-radioactive element A'. The complex has a structure represented by Formula (III): 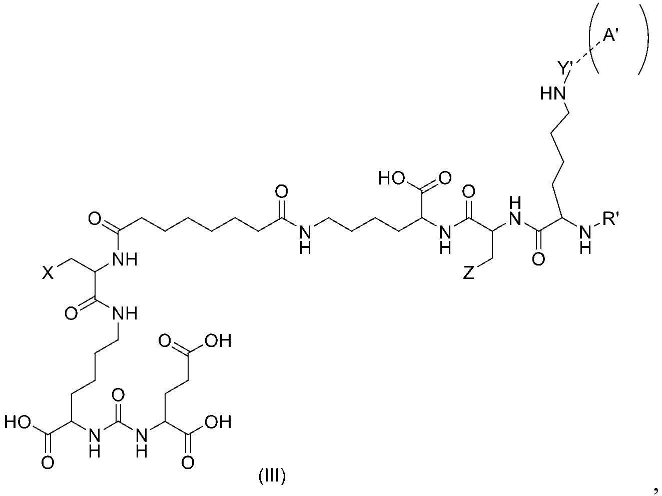 where: Y' is a chelating agent; and R' is H, an optionally substituted amino acid, or an optionally substituted chelating agent.

In still yet another aspect of the present disclosure, the present disclosure also provides a complex. According to the embodiments of the present disclosure, the complex is formed by complexing the compound or the pharmaceutically acceptable salt, ester, or solvate thereof described above with a radionuclide A' or a non-radioactive element A'. The complex has a structure represented by Formula (IV): 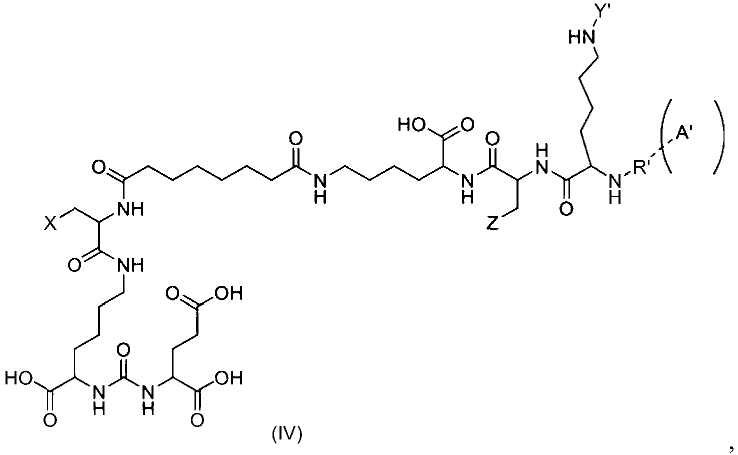 where: R'is a chelating agent; and Y' is H, an optionally substituted amino acid, or an optionally substituted chelating agent.

In still yet another aspect of the present disclosure, the present disclosure also provides a compound, or a pharmaceutically acceptable salt, ester or solvate thereof. The compound has a structure selected from one of: 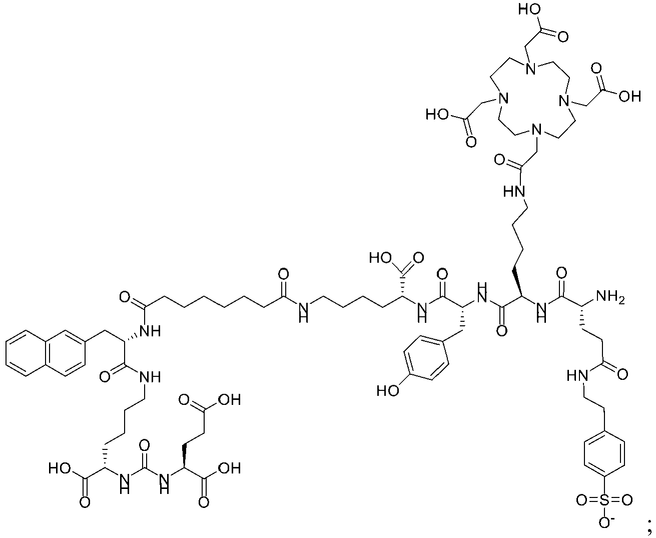 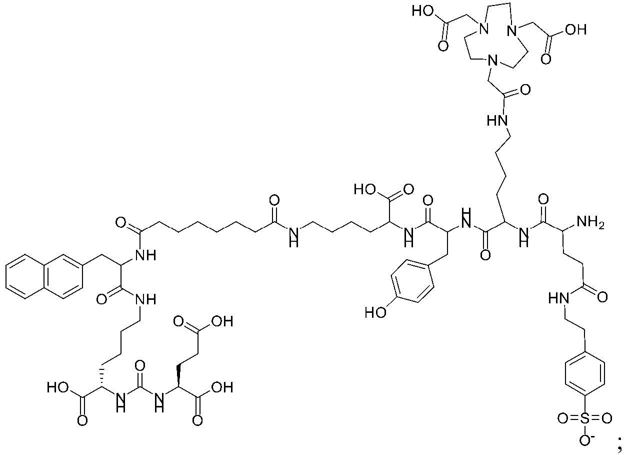 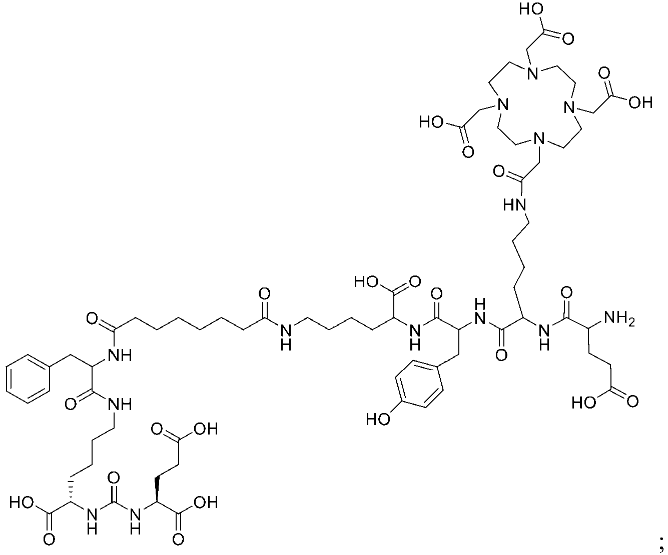 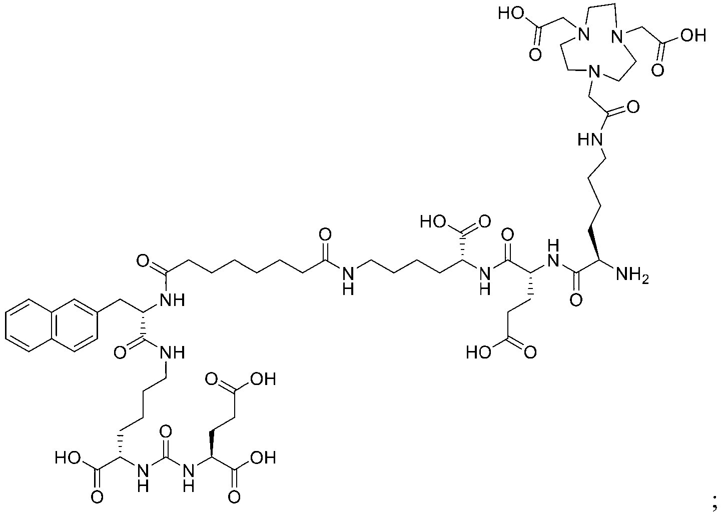 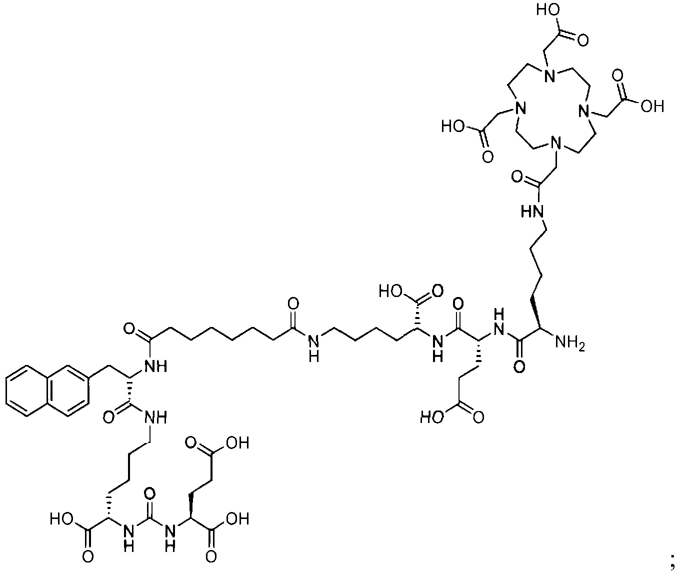 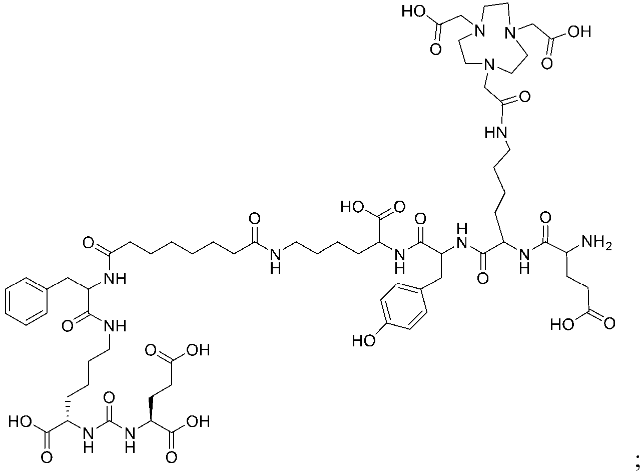 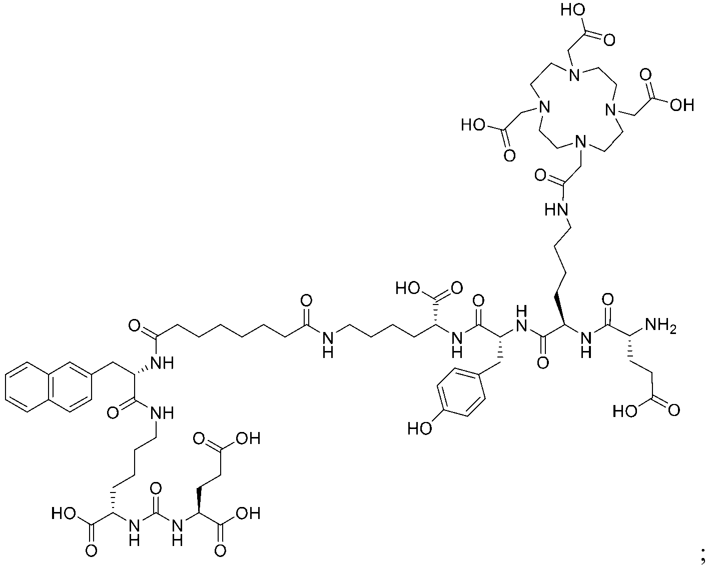 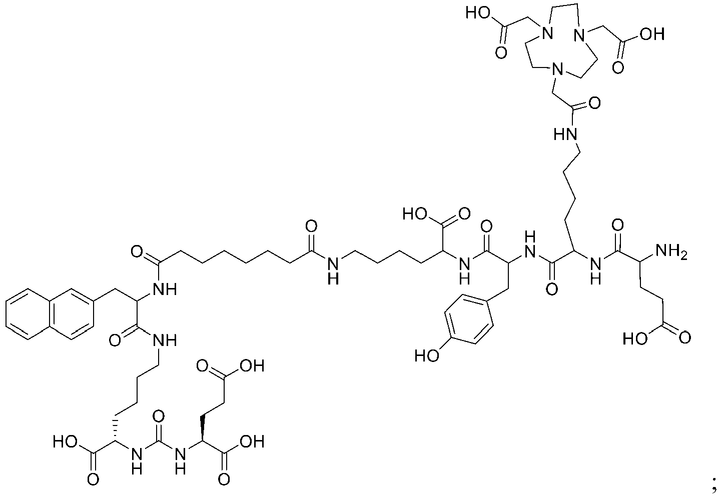 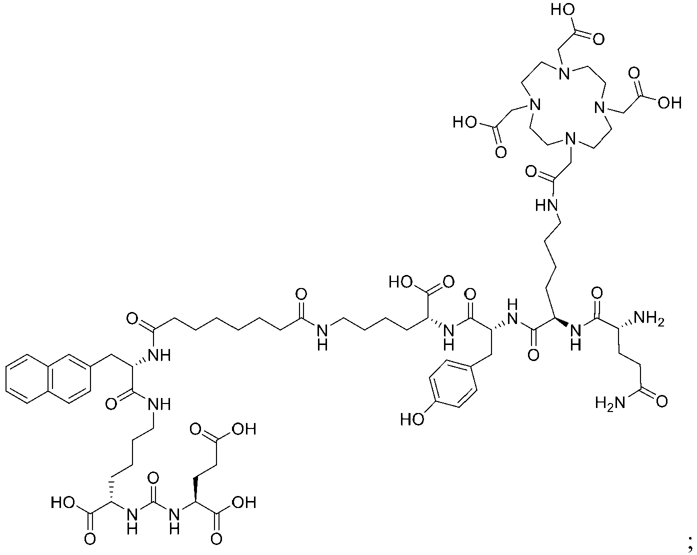 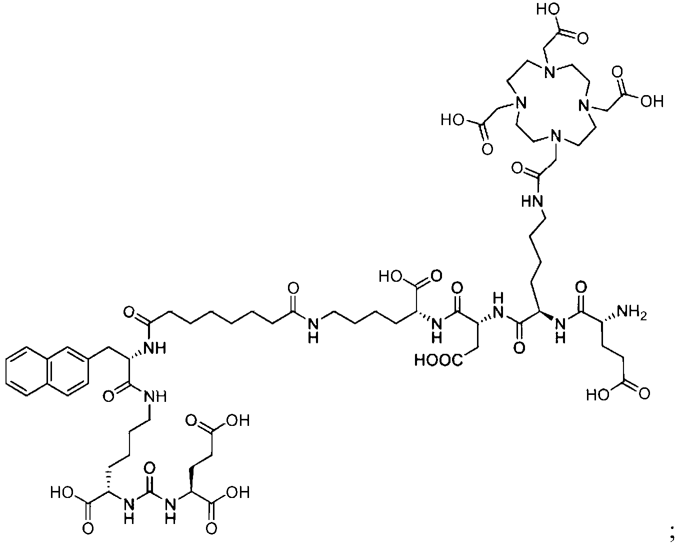 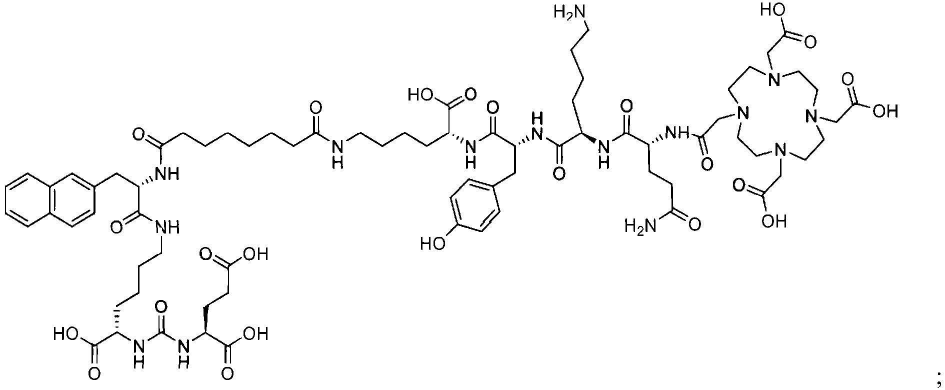 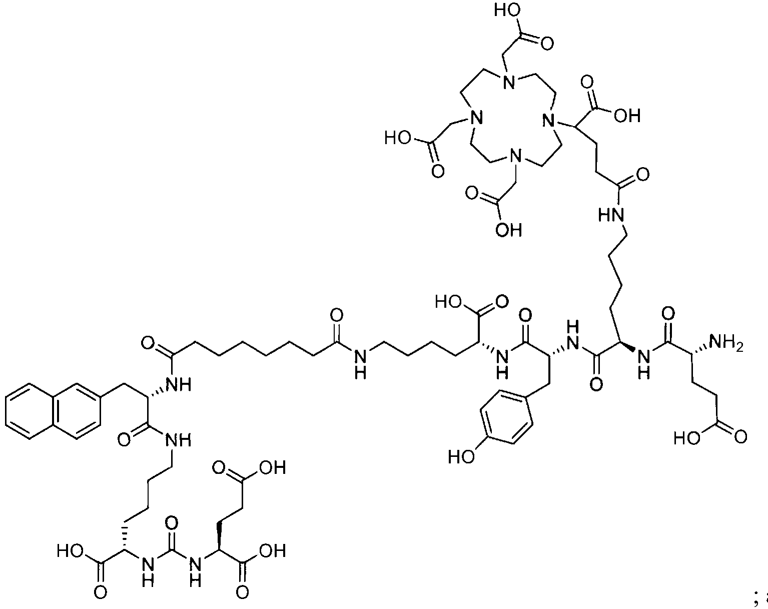 and

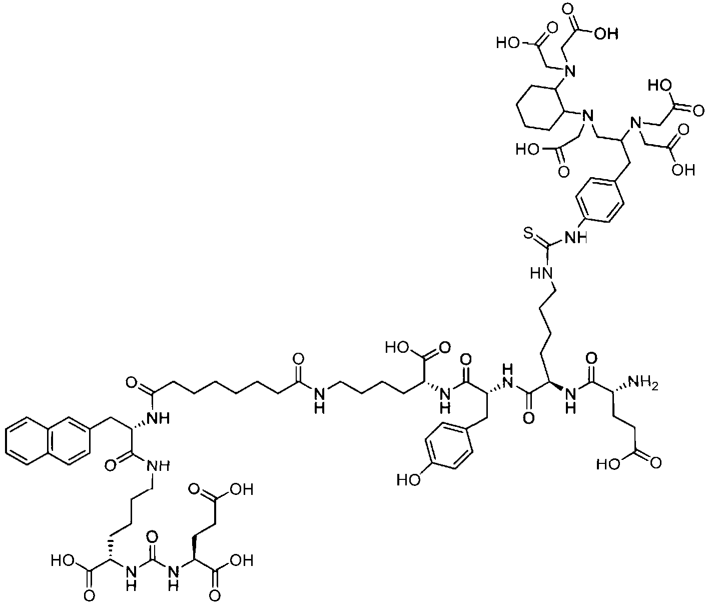

In still yet another aspect of the present disclosure, the present disclosure also provides a complex. According to embodiments of the present disclosure, the complex is formed by complexing the compound or the pharmaceutically acceptable salt, ester, or solvate thereof described above or the complex represented by Formula (III) or Formula (IV) with a radionuclide B' or a non-radioactive element B'.

In some embodiments of the present disclosure, the radionuclide A' or the non-radioactive element A' is same as or different from the radionuclide B' or the non-radioactive element B'.

In some embodiments of the present disclosure, the radionuclide A' or the radionuclide B' is each independently selected from at least one of ⁶⁸Ga, ¹⁸F, ⁹⁹mTc, ⁸⁹Zr, ¹¹¹In, ⁴⁵Ti, ⁵⁹Fe, ⁶⁴Cu, ^{94m}Tc, ⁶⁷Ga, ^{71/72/74}As, ^{43/44}Sc, ^{82m}Rb, ⁵²Mn, ⁸⁶Y, ⁷⁶Br, ¹⁷⁷Lu, ⁹⁰Y, ¹⁵³Sm, ⁶⁷Cu, ⁸⁹Sr, ¹³⁷Cs, ¹⁶⁶Ho, ¹⁷⁷Yb, ¹⁰⁵Rh, ^{186/188}Re, ⁴⁷Sc, ^{212/213}Bi, ²²⁵Ac, ²¹²Pb, ¹⁴⁹Pm, and ²²⁷Th. The remaining variables are as defined in the present disclosure.

In some embodiments of the present disclosure, the non-radioactive element A' or the non-radioactive element B' is each independently selected from at least one of Ga, Fe, and Gd. The remaining variables are as defined in the present disclosure.

In some embodiments of the present disclosure, the radionuclide is selected from ¹⁸F, ⁶⁸Ga, or ¹⁷⁷Lu. The remaining variables are as defined in the present disclosure.

In some embodiments of the present disclosure, the complexation of the radionuclide ¹⁸F is achieved via radioactive aluminum fluoride ([¹⁸F]AlF), preferably, the radioisotope is selected from ¹⁸FAl. The remaining variables are as defined in the present disclosure.

In some embodiments of the present disclosure, the complex has a structure selected from one of: 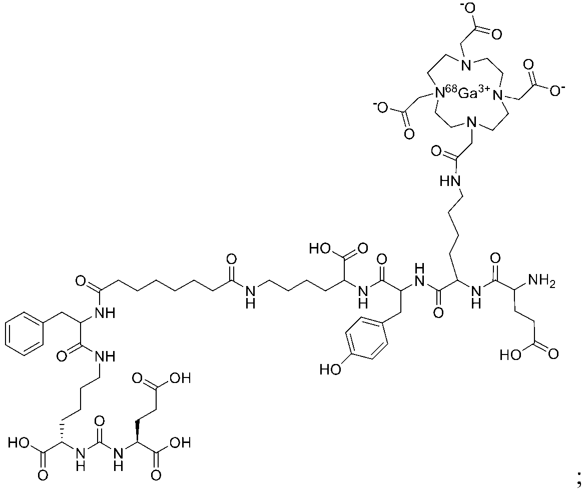 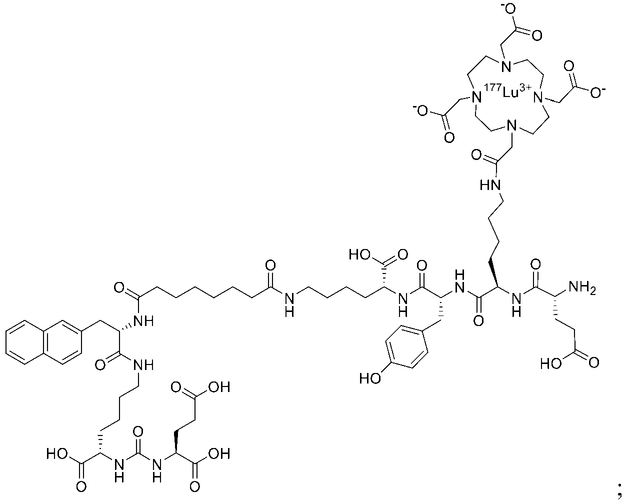 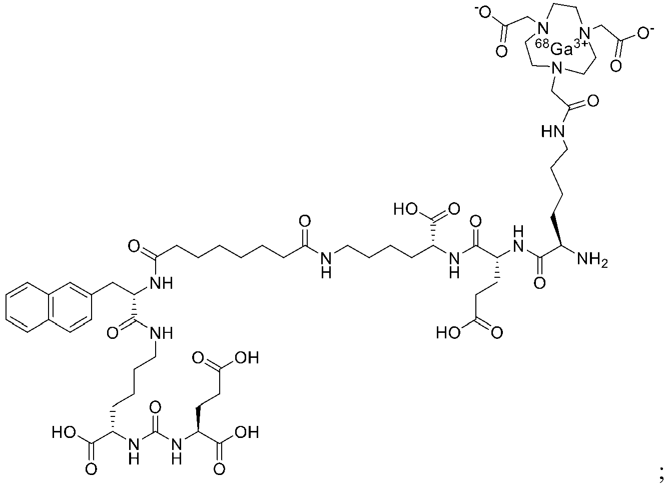 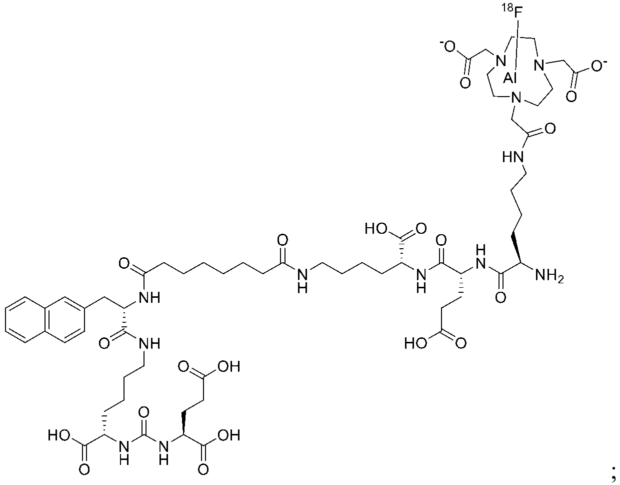 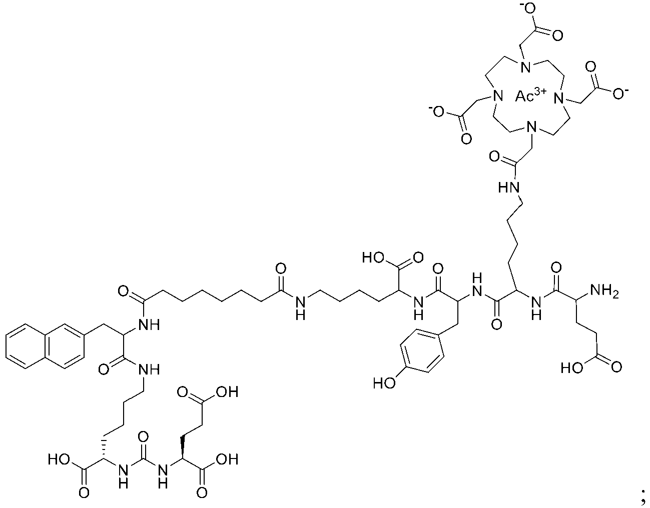 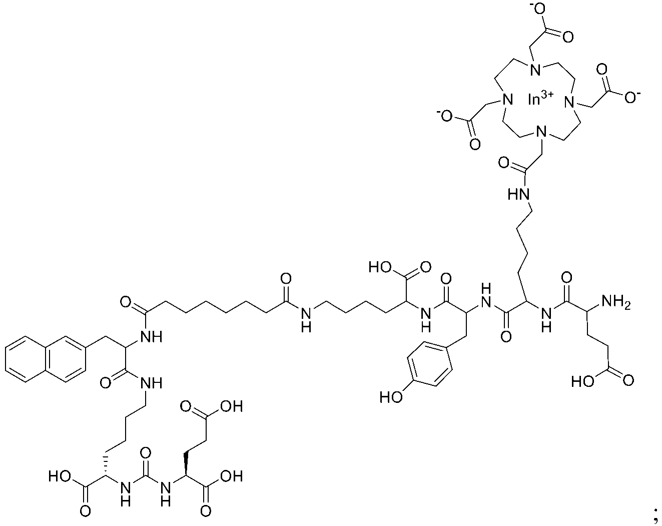 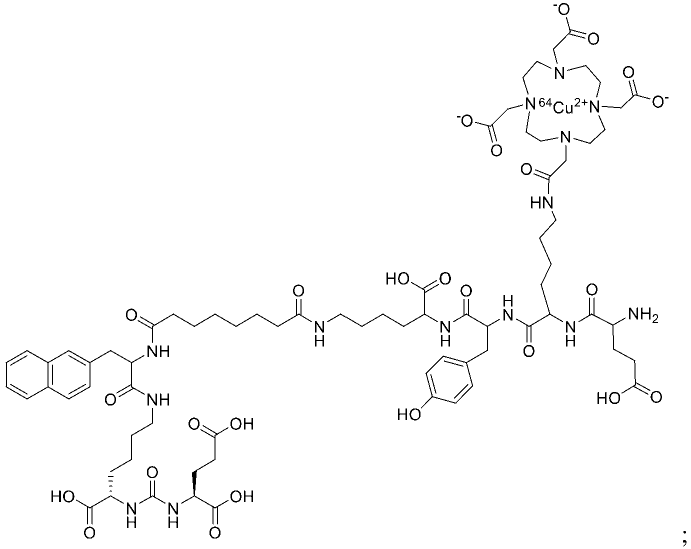 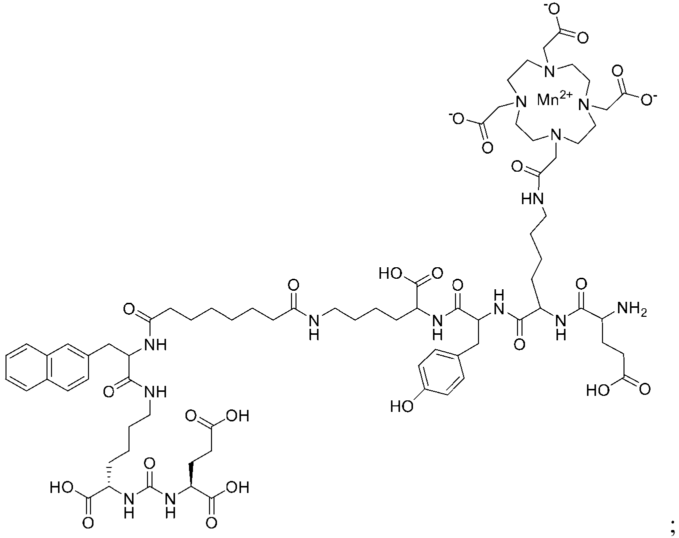 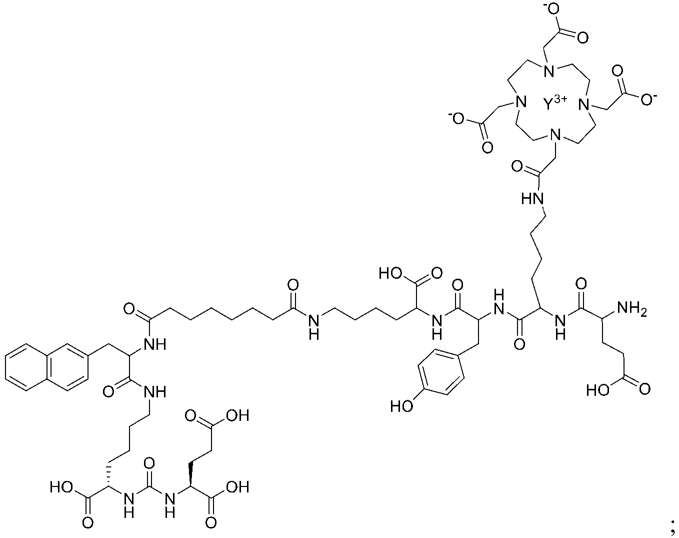 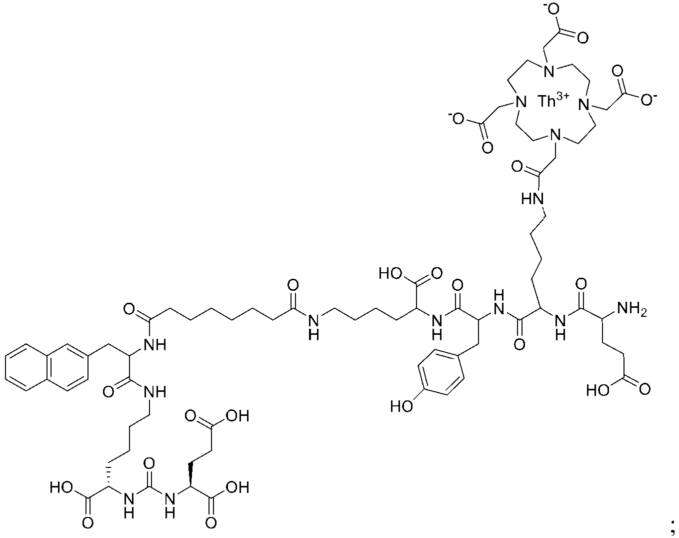 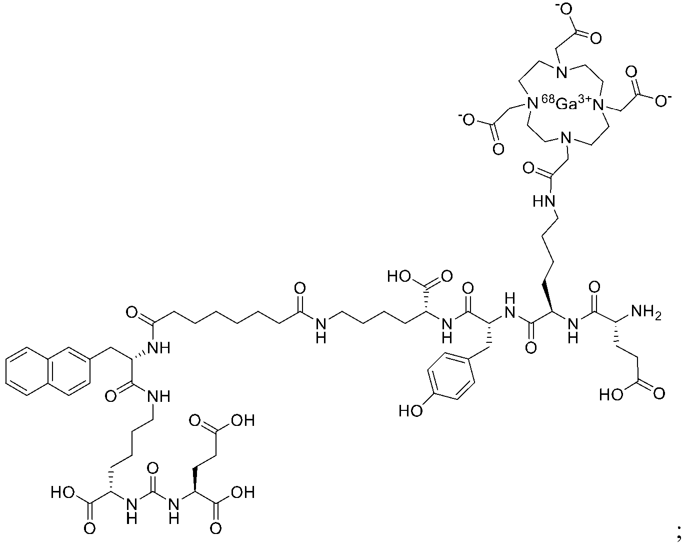 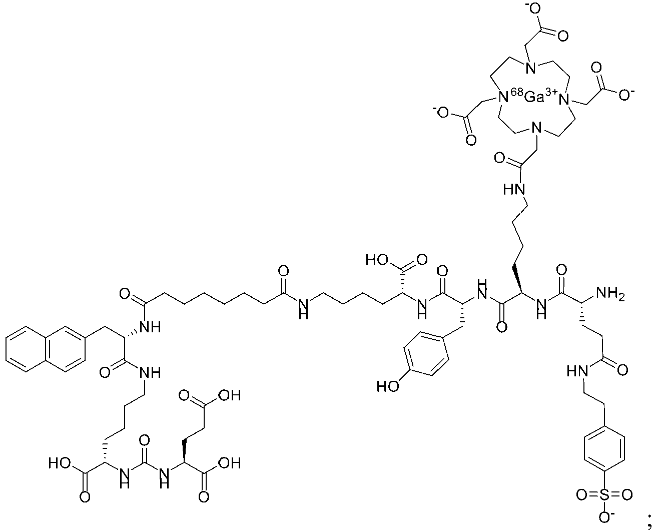 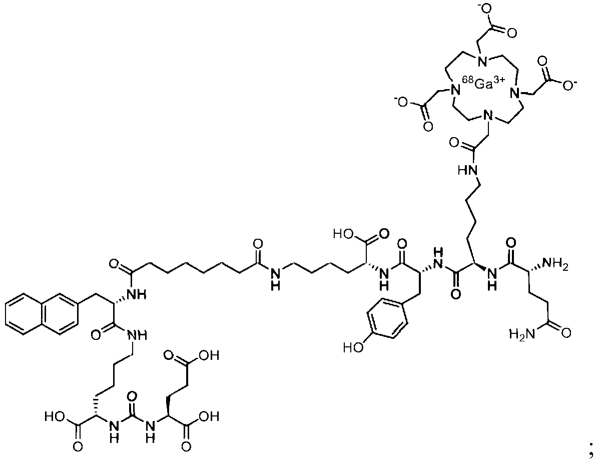 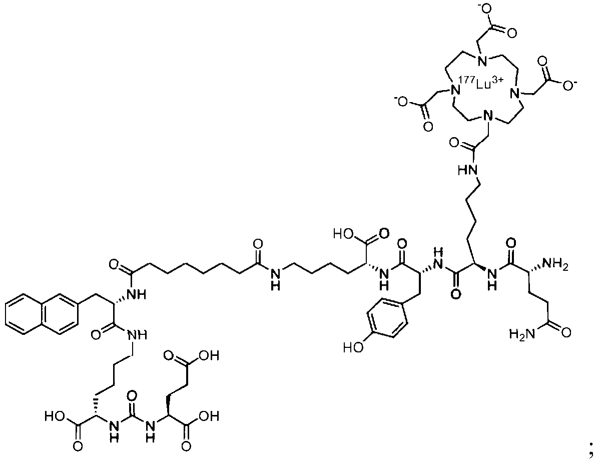 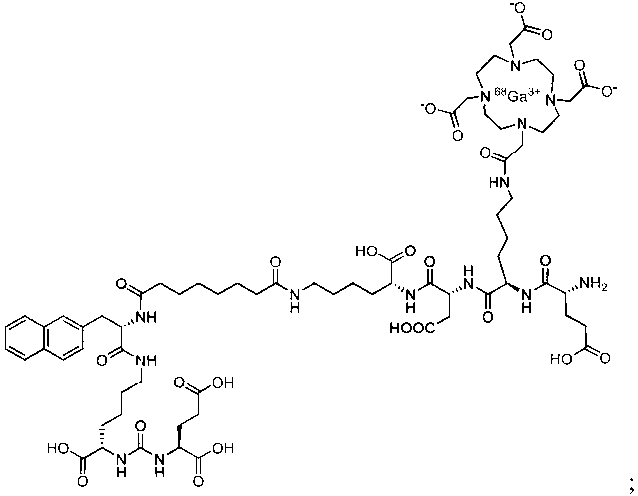 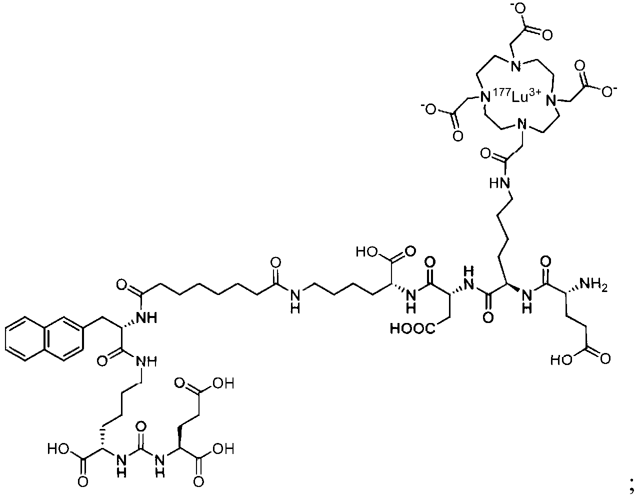 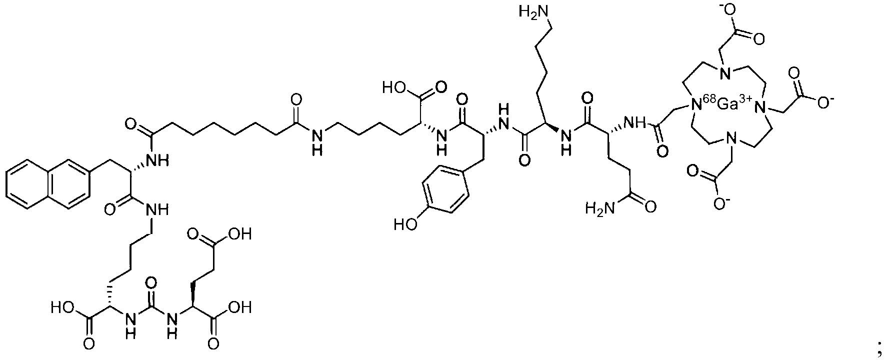 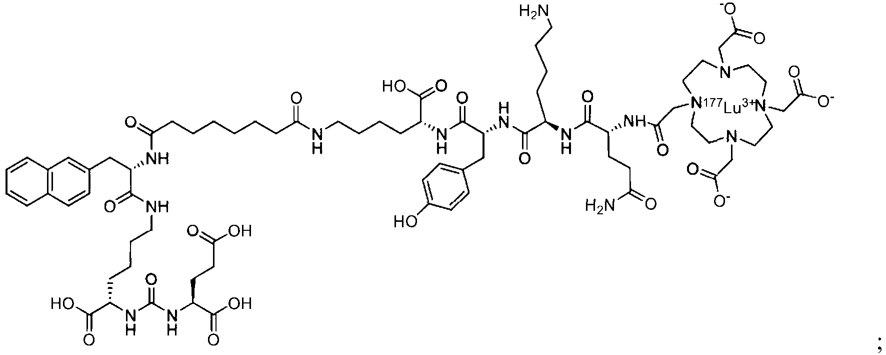 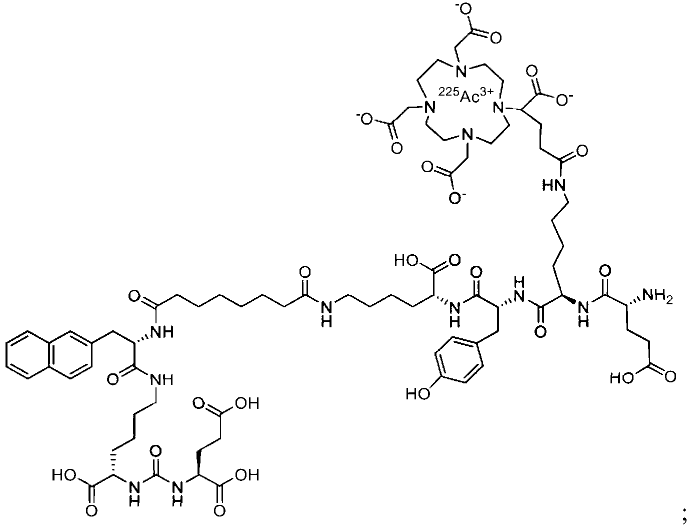 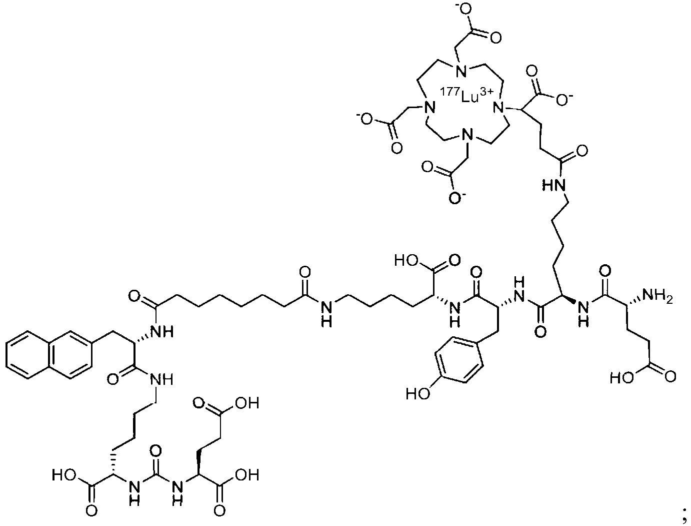 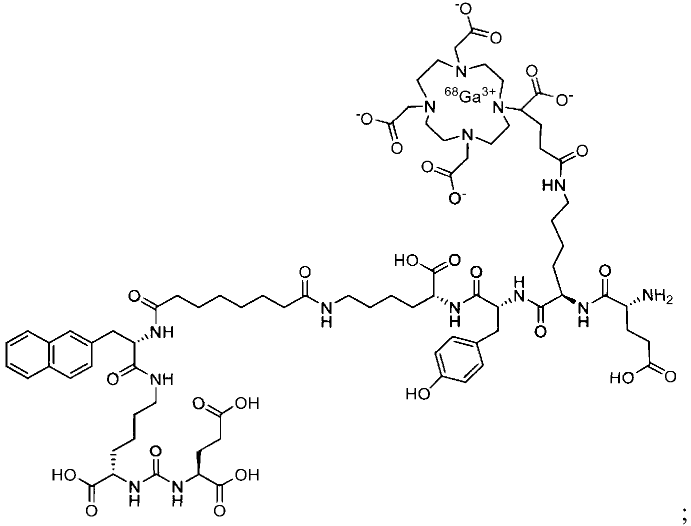 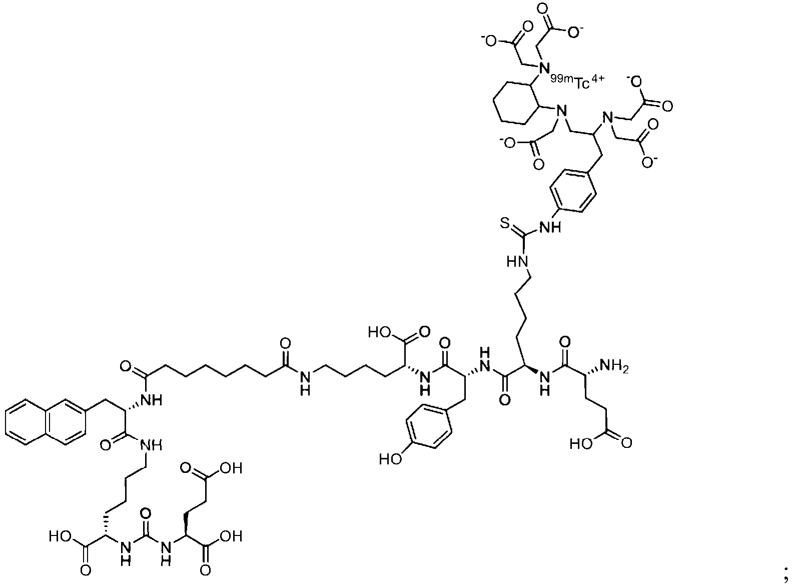 and

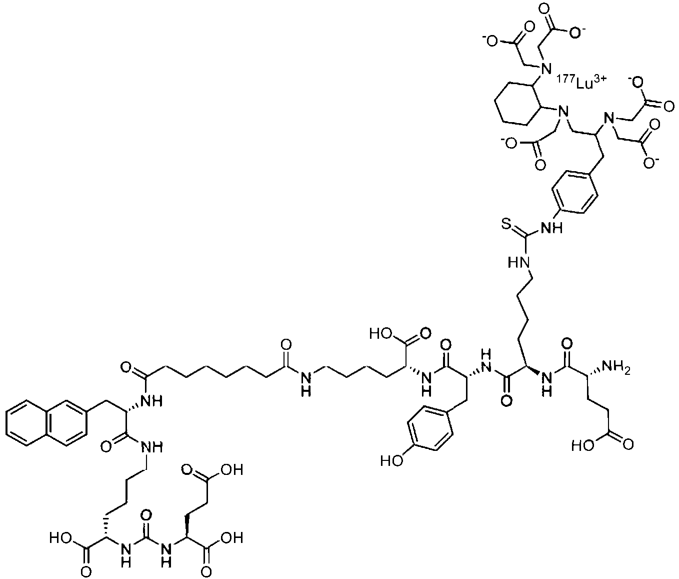

In still yet another aspect of the present disclosure, the present disclosure also provides a pharmaceutical composition. According to embodiments of the present disclosure, the pharmaceutical composition includes: the compound, or the pharmaceutically acceptable salt, ester, or solvate thereof described above, or the complex described above; and a pharmaceutically acceptable carrier or excipient.

In still yet another aspect of the present disclosure, the present disclosure also provides use of the compound, or the pharmaceutically acceptable salt, ester,or solvate thereof described above, the complex described above, or the pharmaceutical composition described above, in the preparation of one or more reagents and/or medicaments for diagnosing and/or treating a tumor, a cancer, or a cell expressing PSMA.

In still yet another aspect of the present disclosure, the present disclosure also provides use of the compound, or the pharmaceutically acceptable salt, ester or solvate thereof described above, the complex described above, or the pharmaceutical composition described above, in diagnosis and/or treatment of a tumor, a cancer, or a cell expressing PSMA.

In still yet another aspect of the present disclosure, the present disclosure also provides a method for diagnosis and/or treatment of a tumor, a cancer, or a cell expressing PSMA. In some embodiments of the present disclosure, the method includes: administering to a patient a pharmaceutically acceptable dose of the compound, or the pharmaceutically acceptable salt, ester, or solvate thereof described above, or the complex described above, or the pharmaceutical composition described above.

In still yet another aspect of the present disclosure, the present disclosure also provides a method for diagnosis and/or treatment of prostate cancer and other solid tumors and/or metastasis thereof. The method includes: using the compound, or the pharmaceutically acceptable salt, ester or solvate thereof described above, or the complex described above, or the pharmaceutical composition described above.

In some embodiments of the present disclosure, the diagnosis is implemented by a means selected from optical imaging and/or radionuclide imaging, and the remaining variables are as defined in the present disclosure. In some embodiments of the present disclosure, the diagnosis is implemented by a means selected from PET imaging and/or SPECT, imaging, and the remaining variables are as defined in the present disclosure.

In some embodiments of the present disclosure, the treatment is selected from radiotherapy, and the remaining variables are as defined in the present disclosure.

In some embodiments of the present disclosure, the cancer is selected from prostate cancer and/or other solid tumors, and the remaining variables are as defined in the present disclosure.

In still yet another aspect of the present disclosure, the present disclosure also provides a kit. According to embodiments of the present disclosure, the kit includes: the compound, or the pharmaceutically acceptable salt, ester, or solvate thereof described above, or the complex described above, and a pharmaceutically acceptable carrier and excipients.

In some embodiments of the present disclosure, the kit further includes a pharmaceutical excipient, and the remaining variables are as defined in the present disclosure.

In some embodiments of the present disclosure, the pharmaceutical excipient includes sterile water for injection, acetic acid/sodium acetate buffer solution, and sodium ascorbate.

In some embodiments of the present disclosure, the kit is prepared by the following method:
1) A precursor solution of the compound or complex described above at a concentration ranging from 0.2 mg/mL to 10 mg/mL is prepared using sterile water for injection as a solvent, and the prepared precursor solution is dispensed into vials at 5 µg to 50 µg per vial.
2) An acetic acid/sodium acetate buffer solution at a concentration ranging from 0.2 mol/L to 0.5 mol/L (a pH value ranging from 3.5 to 4.5) is prepared using sterile water for injection as a solvent, and the prepared acetic acid/sodium acetate buffer solution is dispensed into vials at 0.2 mL to 2 mL per vial.
3) A sodium ascorbate solution at a concentration ranging from 20 mg/mL to 80 mg/mL is prepared using sterile water for injection as a solvent, and the prepared sodium ascorbate solution is dispensed into vials at 0.1 mL to 1 mL per vial.

In some embodiments of the present disclosure, all of the above-mentioned solutions are prepared under a ultra-clean bench with an overall cleanliness of Class C and a local cleanliness class of Class A.

According to the embodiments of the present disclosure, the present disclosure has at least one of the following advantages over the prior arts.
1) In the present disclosure, through the optimization of the molecular structure, a radioactive nuclide chelating group with a relatively large molecular weight is combined with a PSMA target targeting group. According to the current experimental results, there is no significant difference in the efficacy of this molecule in radioactive diagnosis (PET/CT scanning or SPECT/CT scanning) compared with the currently marketed PSMA diagnostic products.
2) A clinical application advantage of the present disclosure is also reflected in the fact that patients are injected with a radioactive labeled drug for the PET/CT scanning or the SPECT/CT scanning, and the imaging results show an extremely high tumor uptake-to-background ratio (a target-to-background ratio). A clinician can obtain data such as a tumor size, a tumor location, and a tumor malignancy through the imaging result.
3) In the present disclosure, through the optimization of the molecular structure, the same molecular structure is enabled to be loaded with both the diagnostic nuclide and the therapeutic nuclide, which are used in pair, thereby truly realizing the theranostics.
4) In the present disclosure, through the optimization of the molecular structure, the drug molecule is enabled to have high affinity and good targeting for the tumor cell, a long retention time at a tumor lesion, rapid clearance from a circulatory system, a long in-vivo half-life, and a high cumulative dose at the tumor lesion. As a result, a single administration dose or an administration frequency is reduced, thereby lowering toxicity.

### Definition and General Term

The present disclosure will list in detail literature corresponding to specific contents, and all embodiments will be accompanied by diagrams of structural and chemical formulas. The present disclosure is intended to cover all alternatives, modifications, and equivalents, which may be included within the scope of the present disclosure as defined by the claims. Those skilled in the art will recognize many methods and substances similar or equivalent to those described herein, which could be applied in the practice of the present disclosure. The present disclosure is by no means limited to the methods and substances described. There are many documents and similar substances that differ from or conflict with the present disclosure, including but not limited to definitions of terms, usage of the terms, described technologies, or the scope controlled by the present disclosure.

The following definitions shall be applied in the present disclosure unless indicated otherwise. For purposes of the present disclosure, chemical elements are defined in accordance with Periodic Table of the Elements, Chemical Abstracts Service, CAS version, and Handbook of Chemical Physics, 75th Ed., 1994. In addition, general principles of organic chemistry are described in: *"*Organic Chemistry" by Thomas Sorrell, University Science Books, Sausalito, 1999, and *"*March's Advanced Organic Chemistry" by Michael B. Smith and Jerry March, John Wiley & Sons, New York, 2007. All of the above-mentioned references are incorporated herein by reference.

As described in the present disclosure, the amino acid refers to an organic compound containing a basic amino group and an acidic carboxyl group.

As described in the present disclosure, the compounds of the present disclosure may be optionally substituted with one or more substituents, such as the above general formula compounds, or as specific examples in the embodiments, subclasses, and classes of compounds encompassed by the present disclosure. It should be understood that the term "optionally substituted" can be used interchangeably with the term "substituted or unsubstituted". In general, the term "optionally", whether preceded by the term "substituted" or not, means that one or more hydrogen atoms in a given structure may be replaced with specified substituents. Unless indicated otherwise, an optionally substituted group may have a substituent at each substitutable position of a group. When more than one position in a given formula can be substituted with one or more substituents selected from a specified group, the substituents may be same or different at each position for substitution. The substituent described herein may be, but is not limited to, deuterium, hydroxyl, amino, halogen, cyano, aryl, heteroaryl, alkoxy, alkylamino, alkylthio, alkyl, alkenyl, alkynyl, heterocyclic, mercapto, nitro, aryloxy, heteroaryloxy, oxo (=O), carboxyl, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyl-substituted alkoxy, 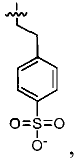 etc.

Unless specified otherwise, the term "alkyl" refers to a saturated linear or branched monovalent hydrocarbon group of 1 to 20 carbon atoms, or 1 to 10 carbon atoms, or 1 to 8 carbon atoms, or 1 to 6 carbon atoms, or 1 to 4 carbon atoms, or 1 to 3 carbon atoms. The alkyl may be independently and optionally substituted with one or more substituents described in the present disclosure. The substituent includes but is not limited to deuterium, amino, hydroxyl, cyano, F, Cl, Br, I, mercapto, nitro, oxo(=O), etc. Examples of the alkyl include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, etc. The term "alkyl" and its prefix "alkane" as used herein include both linear and branched saturated carbon chains. The term "alkylene" as used herein refers to a saturated divalent hydrocarbon radical derived from a linear or branched saturated hydrocarbon by eliminating two hydrogen atoms, and examples of the alkylene include, but are not limited to, methylene, ethylene, isopropylidene, etc.

The term "aryl" may be used alone or as a major part of "aralkyl", "aralkyloxy", or "aryloxyalkyl" to refer to a monocyclic, bicyclic, and tricyclic carbon ring system containing a total of 6 to 14 ring members. At least one of the ring systems is aromatic. Each of the ring systems contains 3 to 7 ring members, and has one or more attachment points connected to the remaining part of the molecule. The term "aryl" may be used interchangeably with the term "aromatic ring". For example, the aromatic ring may include phenyl, naphthyl, and anthracenyl. The aryl may be substituted or unsubstituted, and its substituent may be, but is not limited to, deuterium, hydroxyl, amino, halogen, cyano, aryl, heteroaryl, alkoxy, alkylamino, alkyl, alkenyl, alkynyl, heterocyclic, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyl-substituted alkoxy, etc.

The term "heteroaryl" may be used alone or as a major part of "heteroarylalkyl" or "heteroarylalkoxy" to refer to a monocyclic, bicyclic, and tricyclic ring system containing a total of 5 to 14 ring members. At least one of the ring systems is aromatic and contains one or more heteroatoms. The heteroatom has a meaning described in the present disclosure. Each of the ring systems contains 3 to 7 ring members and has one or more attachment points connected to the rest of the molecule. The term "heteroaryl" may be used interchangeably with the term "heteroaromatic cycle" or "heteroaromatic compound". The heteroaryl may be substituted or unsubstituted, and its substituent may be, but is not limited to, deuterium, hydroxyl, amino, halogen, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyl-substituted alkoxy, etc.

In other embodiments, the aromatic heterocycle includes the following monocyclic rings, but is not limited to these monocyclic rings: 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, 4-methylisoxazol-5-yl, N-pyrrolyl, 2-pyrrolyl, 3-pyrrolyl, 2-pyridyl, 3-pyridyl, 4-pyridyl, 2-pyrimidinyl, 4-pyrimidinyl, pyrimidin-5-yl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thienyl, pyrazolyl (e.g., 2-pyrazolyl ), isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, 1,3,4-thiadiazol-2-yl, pyrazinyl, pyrazin-2-yl, 1,3,5-triazinyl, benzo[d]thiazolyl oxazol-2-yl, or imidazo[1,5-a]pyridin-6-yl. The aromatic heterocycle further includes the following bicyclic rings, but is by no means limited to these bicyclic rings: benzimidazolyl, benzofuranyl, benzothiophenyl, indolyl (e.g., 2-indolyl), purinyl, quinolyl (e.g., 2-quinolyl, 3-quinolyl, 4-quinolyl), and isoquinolyl (e.g., 1-isoquinolyl, 3-isoquinolyl or 4-isoquinolyl).

The term "halogen" refers to F, Cl, Br, and I.

In addition, it should be noted that unless explicitly stated otherwise, the descriptive phrases "each... independently " and... and... each independently" throughout this specification can be used interchangeably and should be understood broadly. It can mean that in different groups, the specific options represented by the same symbols do not affect each other, or in the same group, the specific options represented by the same symbols do not affect each other. For example, in a structure( 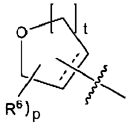 and a structure 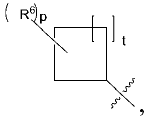 specific options of R⁶ in the two structures are independent of each other. Meanwhile, when a plurality of R⁶ appear in a same structure, specific options of the plurality of R⁶ do not affect each other. That is, the specific options of R⁶ can be the same or different.

In the structural formula of the compound or the ligand described in the present disclosure, the bond "_" indicates that the configuration is unspecified. If chiral isomerism exists in the chemical structure, the bond "_" can be a configuration of " 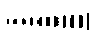 " or " 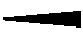 ", or contain the configurations of both " 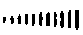 " and " 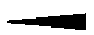 ". Although all of the above structural formulae are drawn in certain isomeric forms for simplicity, the present disclosure may include all isomers, such as tautomers, rotamers, geometric isomers, diastereomers, racemates, and enantiomers.

Definitions and conventions for stereochemistry used in the present disclosure are generally based on the following references: SP Parker, Ed., McGraw-Hill Dictionary of Chemical Terms (1984) McGraw-Hill Book Company, New York; and Eliel, E. and Wilen, S., "Stereochemistry of Organic Compounds", John Wiley&Sons, Inc., New York, 1994. The compounds of the present disclosure may contain an asymmetric or chiral center and therefore different stereoisomers exist. All stereoisomeric forms of the compounds of the present disclosure, including but by no means limited to, diastereomers, enantiomers, atropisomers, and mixtures thereof, such as racemic mixtures, constitute a part of the present disclosure. Many organic compounds exist in optically active forms, that is, they have an ability to rotate a plane of plane-polarized light. When describing an optically active compound, prefixes D and L or R and S are used to denote an absolute configuration of chiral center of a molecule. Prefixes d and l or (+) and (-) are signs used to name the rotation of plane-polarized light of the compound. (-) or l means that the compound is levorotatory, and the prefix (+) or d means that the compound is dextrorotatory. These stereoisomers have the same chemical structures but differ in their stereo-structures. A specific stereoisomer may be a mixture of enantiomers or isomers, and is commonly referred as to an enantiomeric mixture. A 50:50 mixture of enantiomers is referred as to a racemic mixture or racemate, which may lead to a lack of stereoselectivity or stereospecificity during a chemical reaction. The terms "racemic mixture" and "racemate" refer to an equimolar mixture of two enantiomers, devoid of optical activity.

The term "tautomer" or "tautomeric form" refers to structural isomers of different energies that are interconvertible through a low energy barrier. For example, a proton tautomer (i.e., a prototropic tautomer) includes interconversion through proton migration, such as keto-enol and imine-enamine isomerization. A valence (chemical valence) tautomer includes interconversion through reorganization of a bonding electron.

The term "pharmaceutically acceptable salt" used in the present disclosure refers to organic and inorganic salts of the compounds of the present disclosure. The pharmaceutically acceptable salt is well-known in the art, as recorded in literatures, such as S.M. Berge et al., J. Pharmaceutical Sciences, 66, 1-19, 1977. A pharmaceutically acceptable salt formed by non-toxic acid includes, but is not limited to, an inorganic acid salt formed by reaction with an amino group, such as hydrochloride, hydrobromide, phosphate, sulfate, perchlorate, and an organic acid salt, such as acetate, oxalate, maleate, tartrate, citrate, succinate, malonate, or the salts obtained by other methods documented in a book and literature, such as ion exchange methods. Other pharmaceutically acceptable salts include adipate, alginate, ascorbate, aspartate, benzenesulfonate, benzoate, bisulfate, borate, butyrate, camphorate, camphorsulfonate, cyclopentylpropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, etc. A salt derived from an appropriate base includes alkali metal, alkaline earth metal, ammonium, and N⁺(C₁₋₄ alkyl)₄ salts. The present disclosure also contemplates a quaternary ammonium salt formed by any compound containing an N group. A water-soluble or oil-soluble or dispersible product can be obtained by quaternization. A alkali metal or alkaline earth metal salt includes sodium, lithium, potassium, calcium, magnesium, etc. A pharmaceutically acceptable salt further includes an appropriate, non-toxic ammonium/quaternary ammonium salt and an amine cation formed with a counterion, such as halide, hydroxide, carboxylate, sulfate, phosphate, nitrate, C₁₋₈ sulfonate, and aromatic sulfonate.

The term "solvate" as used in the present disclosure refers to an associative compound formed by one or more solvent molecules and the compound of the present disclosure. A solvent for forming the solvate includes, but is not limited to, water, isopropanol, ethanol, methanol, dimethyl sulfoxide, ethyl acetate, acetic acid, and aminoethanol. The term "hydrate" refers to an associative compound in which a solvent molecule is water.

The following abbreviations are used throughout the present disclosure:

DOTA represents 1,4,7,10-tetraazacyclododecane-N,N',N",N"',-tetraacetic acid, NOTA represents 1,4,7-triazacyclononane-1,4,7-triacetic acid, NODAGA represents 2-(4,7-bis(carboxymethyl)-1,4,7-triazanonan-1-yl)pentanedioic acid, DOTAGA represents 2-(4,7,10-tris(carboxymethyl)-1,4,7,10-tetraazacyclododecan-1-yl)pentanedioic acid, TRAP represents 1,4,7-triazacyclononane phosphinic acid, NOPO represents 1,4,7-triazacyclononane-1-[methyl(2-carboxyethyl)phosphinic acid]-4,7-bis[methyl(2-carboxymethyl)phosphinic acid], DFO represents N'-{5-[acetyl(hydroxy)amino]pentyl}-N-[5-({4-[5-aminopentyl)(hydroxy)amino]-4-oxobutanoyl}amino)pentyl]-N-hydroxysuccinamide, DTPA represents diethylenetriaminepentaacetic acid, CHX-DTPA represents trans-cyclohexyl-diethylenetriaminepentaacetic acid, SCN-Bz-DTPA represents p-isothiocyanatobenzyl-DTPA, 1B3M represents 1-(isothiocyanatobenzyl)-3-methyl-DTPA, 1B3B represents 1-(isothiocyanatobenzyl)-4-methyl-DTPA, MX-DTPA represents 1-(2)-methyl-4-isothiocyanatobenzyl-DTPA, oxo-Do3A represents 1-oxa-4,7,10-triazacyclododecane-4,7,10-triacetic acid, HYNIC represents 6-hydrazinonicotinic acid succinimidyl ester hydrochloride; p-SCN-Bn-CHX-A"-DTPA represents [(R)-2-amino-3-(4-isothiocyanatophenyl)propyl]-trans-(S,S)-cyclohexane-1,2-diaminopentaacetic acid; and MAG3 represents mercaptoacetyltriglycine.

### BRIEF DESCRIPTION OF THE DRAWINGS

The foregoing and/or additional aspects and advantages of the present disclosure will become apparent and readily appreciated from the following description of the examples in conjunction with the accompanying drawings, of which:
FIG. 1 is a LC-MS spectrum of NYM032 according to an embodiment of the present disclosure.
FIG. 2 is a HPLC spectrum of NYM032 according to an embodiment of the present disclosure.
FIG. 3 is a purity analysis graph of a ⁶⁸Ga-NYM032 by a radioactive thin-layer chromatography scanning according to an embodiment of the present disclosure.
FIG. 4 is a body weight graph of ICR mice after administration according to an embodiment of the present disclosure.
FIG. 5 is a PET/CT imaging result of ⁶⁸Ga-NYM032 on a LNcap tumor-bearing mouse model according to an embodiment of the present disclosure, where the arrow indicates a tumor site.
FIG. 6 is an LC-MS spectrum of NYM033 according to an embodiment of the present disclosure.
FIG. 7 is a HPLC spectrum of NYM033 according to an embodiment of the present disclosure.
FIG. 8 is a purity analysis graph of ⁶⁸Ga-NYM033 by a radioactive thin-layer chromatography scanning according to an embodiment of the present disclosure.
FIG. 9 is a PET/CT imaging results of ⁶⁸Ga-NYM033 on a LNcap tumor-bearing mouse model according to an embodiment of the present disclosure, where an arrow indicates a tumor site.
FIG. 10 is a competitive inhibition experiment scanning image of ⁶⁸Ga-NYM032 and ⁶⁸Ga-NYM033 on a LNcap tumor-bearing mouse model according to an embodiment of the present disclosure, where A and B are ⁶⁸Ga-NYM032 and ⁶⁸Ga-NYM033, respectively, and an arrow indicates a tumor site.
FIG. 11 is a PET/CT imaging results of ⁶⁸Ga-NYM032 in a clinical research trial according to an embodiment of the present disclosure.
FIG. 12 is a PET/CT imaging results of ⁶⁸Ga-NYM033 in a clinical research trial according to an embodiment of the present disclosure.
FIG. 13a is a PET/CT imaging results of patient after administration of ⁶⁸Ga-NYM032 and ⁶⁸Ga-PSMA11 for 1 hour in a clinical research trial according to an embodiment of the present disclosure.
FIG. 13b is a PET/CT imaging results of patient after administration of ⁶⁸Ga-NYM032 and ⁶⁸Ga-PSMA11 for 2 hours in a clinical research trial according to an embodiment of the present disclosure.
FIG. 14a is a PET/CT imaging results of patient after administration of ⁶⁸Ga-NYM032 and ⁶⁸Ga-PSMA617 for 1 hour in a clinical research trial according to an embodiment of the present disclosure.
FIG. 14b is a PET/CT imaging results of patient after administration of ⁶⁸Ga-NYM032 and ⁶⁸Ga-PSMA617 for 2 hours in a clinical research trial according to an embodiment of the present disclosure.
FIG. 15 is a TLC graph of ¹⁷⁷Lu-NYM032 according to an embodiment of the present disclosure.
FIG. 16 is a tumor volume trend graph on a tumor-bearing mouse model respectively treated with ¹⁷⁷Lu-NYM032 and saline according to an embodiment of the present disclosure.
FIG. 17 is a body weight trend graph of a tumor-bearing mouse respectively treated with ¹⁷⁷Lu-NYM032 and saline according to an embodiment of the present disclosure.
FIG. 18 is a PET/CT imaging results of a patient after 1 hour administration of ⁶⁸Ga-NYM032 in a clinical research trial according to an embodiment of the present disclosure.
FIG. 19 is a SPECT/CT imaging results of a patient at 0.5 hours, 1 hour, 24 hours, 48 hours, 120 hours, and 192 hours after administration of ¹⁷⁷Lu-NYM032 in a clinical research trial according to an embodiment of the present disclosure.
FIG. 20 is a SPECT/CT imaging results of a patient at 336 hours and 504 hours after administration of ¹⁷⁷Lu-NYM032 in a clinical research trial according to an embodiment of the present disclosure.
FIG. 21 is a pharmacokinetic curve graph of a patient after administration of ¹⁷⁷Lu-NYM032 in a clinical research trial according to an embodiment of the present disclosure.
FIG. 22 is a graph showing a relationship between radioactive activity in lesions in vivo and time after administration of ¹⁷⁷Lu-NYM032 to a patient in a clinical research trial according to an embodiment of the present disclosure.

### DETAILED DESCRIPTION OF THE EMBODIMENTS

The embodiments of the present disclosure are described below in combination with specific examples. It will be understood by those skilled in the art that the following examples are only used to illustrate the present disclosure and should not be construed as a limitation to the scope of the present disclosure. Where specific techniques or conditions are not specified in the examples, they are performed according to techniques or conditions described in the literature in the art or according to the product description. Reagents and instruments used without specifying the manufacturer are all conventional and commercially available products.

### Example 1 Preparation process of compound NYM032

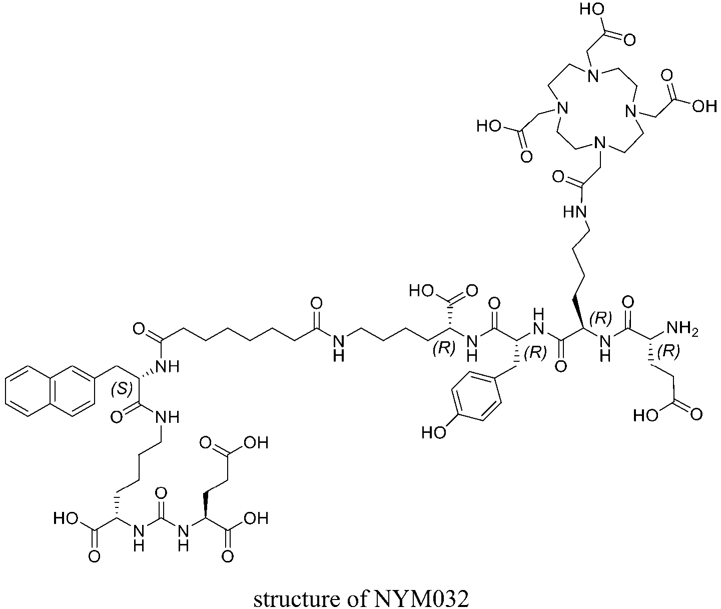

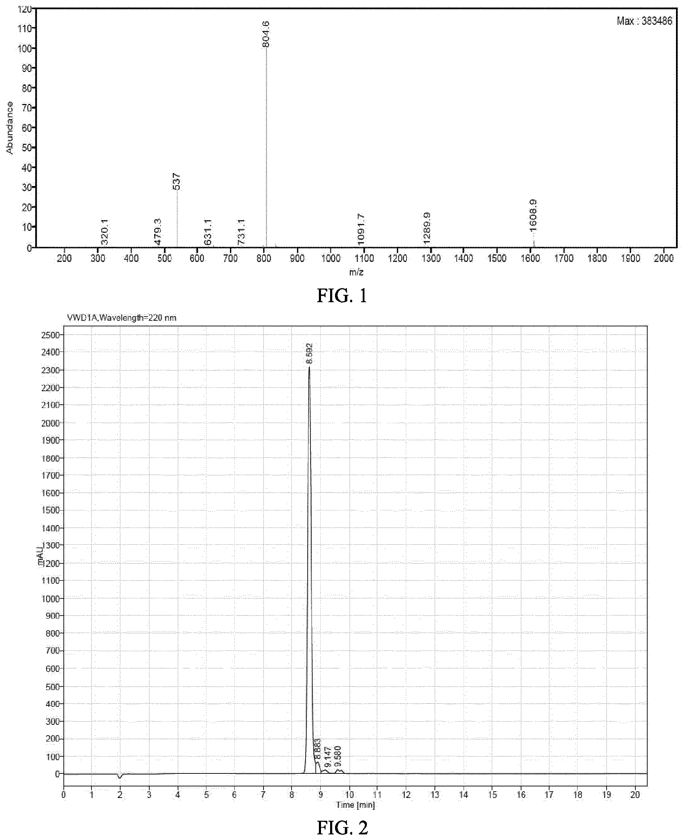

The LC-MS spectrum is shown in FIG. 1, and the HPLC spectrum is shown in FIG. 2.

### Synthesis route:

### Step 1:

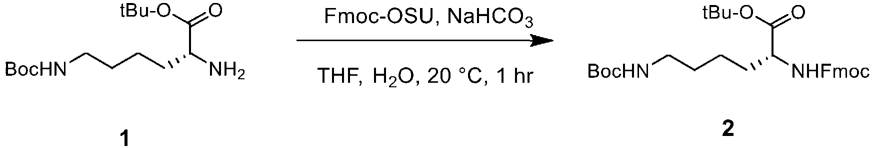

Compound (1) (10.0 g, 29.5 mmol, 1.00 eq, HCl) was dissolved in a mixed solution of THF (100 mL) and H₂O (100 mL), and Fmoc-Osu (9.46 g, 28.0 mmol, 0.95 eq) and NaHCO₃ (7.44 g, 88.5 mmol, 3.44 mL, 3.00 eq) were added and stirred at 20°C for 1 hour. Thin layer chromatography (petroleum ether: ethyl acetate=1:1) indicated that a new compound was produced. The reaction mixture was diluted with 500 mL of H₂O and extracted with ethyl acetate for 3 times, using 500mL of ethyl acetate each time. An organic layer was washed with 500 mL of brine, dried over Na₂SO₄, filtered, and concentrated under reduced pressure to obtain a crude product. The obtained crude product was ground with MTBE (30.0 mL) at 20°C for 2 hours to obtain Compound (2) (14.2 g, 27.0 mmol, a 91.7% yield) as a white solid.

### Step 2:

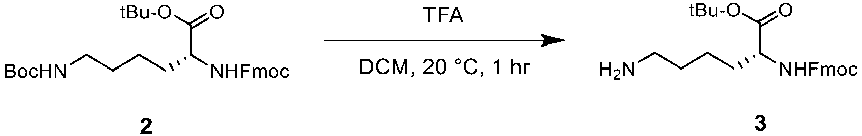

Compound (2) (13.0 g, 24.7 mmol, 1.00 eq) was dissolved in DCM (90.0 mL), TFA (46.2 g, 405 mmol, 1.00 eq) was added and stirred at 20°C for 1 hour, and a NaHCO₃ solution was added to adjust a pH value to 8. The mixture was extracted with ethyl acetate for 3 times, using 500mL of ethyl acetate each time. An organic layer was washed with 200 mL of concentrated brine, dried over Na₂SO₄, filtered, and concentrated under reduced pressure to obtain a yellow oil containing a crude product of Compound (3).

### Step 3:

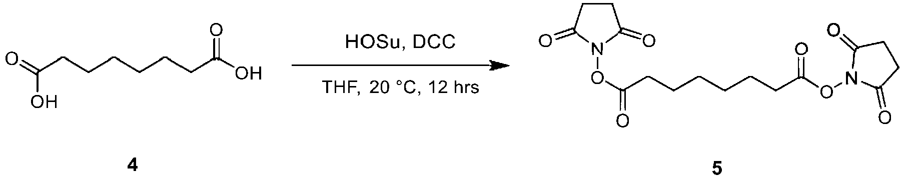

Compound (4) was dissolved in THF (500 mL), DCC (26.0 g, 126 mmol, 1.00 eq) and HOSu (14.5 g, 126 mmol, 2.20 eq) were added, and the mixture was stirred at 20° C for 12 hours to obtain a reaction mixture. The obtained reaction mixture was filtered and concentrated under reduced pressure to obtain 12.0 g of a white solid containing Compound (5).

### Step 4:

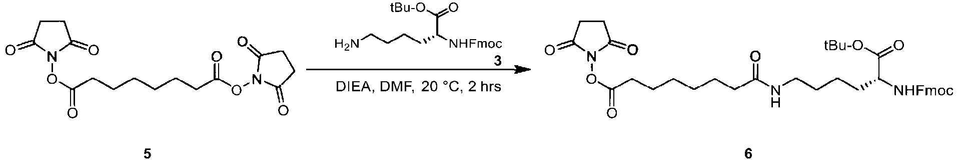

Compound (5) (12.0 g, 32.5 mmol, 1.00 eq) was dissolved in DMF (100 mL) and DIEA (12.6 g, 97.7 mmol, 17.0 mL, 3.00 eq) was added. A DMF (100 mL) solution containing Compound (3) (6.92 g, 16.2 mmol, 0.5 eq) was added dropwise to the above solution and stirred at 20°C for 2 hours. The mixture was diluted with 500 mL of water. An organic layer was washed with 500 mL of concentrated brine, dried over Na₂SO₄, filtered, and concentrated under reduced pressure, to obtain a crude product. The obtained crude product was purified through preparative high-performance liquid chromatography, to obtain Compound (6) as a yellow oil (2.90 g, 4.28 mmol).

### Step 5:

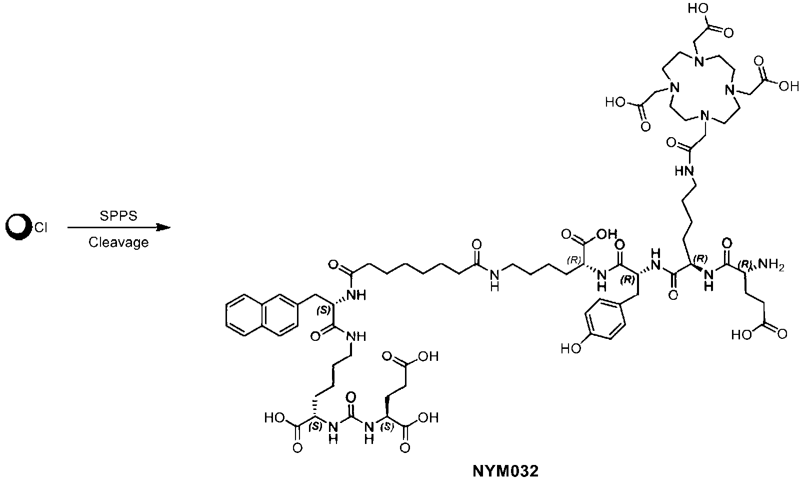

Synthesis of peptide: the peptide was synthesized using a Fmoc synthesis method.
1. Preparation of resin: 2-CTC (0.10 mmol, 1.00 mmol, Sub 0.50 mmol/g) and Fmoc-Lys(Dde)-OH (0.05 g, 0.10 mmol, 1.00 eq) were added into a solid-phase reactor, DCM (10.0 mL) was added, and the mixture was bubbled with N₂. Then, DIEA (0.05 g, 0.40 mmol, 0.07 mL, 4.00 eq) was added dropwise, the mixture was reacted at room temperature (20°C) with N₂ bubbling for 2 hours. MeOH (0.20 mL) was added, and the mixture was continuously stirred for 30.0 minutes. Subsequently, a resin was washed with DMF for 5 times, using 50 mL of DMF each time, to obtain a resin.
2. 50 mL of a DMF solution containing 20% piperidine was added into the reactor, the mixture was reacted with N₂ bubbling for 30.0 minutes, and the resin was washed with DMF five times.
3. Condensation: H-Glu(OtBu)-OtBu.HCl (0.09 g, 0.30 mmol, 3.00 eq), CDI (0.05 g, 0.30 mmol, 3.00 eq), and DMAP (0.04 g, 0.30 mmol, 3.00 eq) were dissolved in THF (200.0 mL). Then, DIEA (0.03 g, 0.20 mmol, 0.04 mL, 2.00 eq) was added dropwise, and the mixture was reacted at 20°C for 1 hour. Then, this solution was added to the resin, and the mixture was stirred at 20°C for 12 hours with N₂ bubbling. Then, the resin was washed with DMF for 5 times, using 50 mL of DMF each time.
4. 3% hydrazine hydroxide (50.0 mL) was added to the resin, and the mixture was continuously reacted with N₂ bubbling for 1 hour. Then, the resin was washed with DMF for 5 times, using 50 mL of DMF each time, and filtered.
5. Condensation: Fmoc-2-Nal-OH (0.07 g, 0.15 mmol, 1.50 eq) and HBTU (0.05 g, 0.14 mmol, 1.43 eq) were dissolved in 10.0 mL DMF, the solution was added to the resin, and the mixture was bubbled with N₂. Then, DIEA (0.04 g, 0.30 mmol, 0.05 mL, 3.00 eq) was added to the mixture, and the mixture was reacted at 20°C with N₂ bubbling for 30.0 minutes. Then, the resin was washed with DMF for 5 times, using 50 mL of DMF each time.
6. A 50 mL of DMF solution containing 20% piperidine was added into the resin, and the mixture was continuously reacted with N₂ bubbling for 30.0 minutes. Then, the resin was washed with DMF for 5 times, using 50 mL of DMF each time, and filtered, to obtain a resin.
7. Steps 5 and 6 above were repeated to sequentially couple amino acid in Table 1 (serial numbers c to h):

**[Table 1]**

| **Serial number** | **Coupling amino acid** | **Coupling reagent** |
|---|---|---|
| a | Fmoc-Lys(Dde)-OH (1.00 *eq*) | DIEA (4.00 *eq*) |
| b | H-Glu(OtBu)-OtBu.HCl (3.00 *eq*) | CDI (3.00 *eq*), DMAP (3.00 *eq*), DIEA (2.00 *eq*) |
| c | Fmoc-2-Nal-OH (1.50 *eq*) | HOAT (1.43 *eq*), DIC (1.50 *eq*) |
| d | Compound (6) (1.50 *eq*) | DIEA (3.00 *eq*) |
| e | Fmoc-D-Tyr(tBu)-OH (3.00 *eq*) | HOAT (2.85 *eq*), DIC (3.00 *eq*) |
| f | Dde-D-Lys(Fmoc)-OH (1.50 *eq*) | HOAT (1.43 *eq*), DIC (1.50 *eq*) |
| g | DOTA-tri(t-butyl ester) (2.00 *eq*) | HBTU (1.90 *eq*), DIEA (4.00 *eq*) |
| h | Fmoc-D-Glu(tBu)-OH (1.50 *eq*) | HOAT (1.43 *eq*), DIC (1.50 *eq*) |

8. A DMF solution containing 20% piperidine was added to the resin, and the mixture was continuously stirred for 30.0 minutes to remove an Fmoc protecting group. The deprotection reaction was monitored with a ninhydrin detection reagent, and the resin was washed five times with DMF after the reaction was completed.
9. Cleavage and purification of peptide: a peptide with a side-chain protecting group and a cleavage solution (90% TFA, 2.5% H₂O, 2.5% 3-Mercaptopropionic acid, 5% TIS) were added into a flask and stirred at room temperature for 2 hours for deprotection. The mixture was precipitated with tert-butyl methyl ether and placed at -20°C for 5.0 minutes, and filtered to collect a filter cake. Then, the filter cake was washed with tert-butyl methyl ether twice and was vacuum dried for 2 hours. The crude product was purified by using preparative high-performance liquid chromatography (A: an aqueous solution of 0.075% TFA, B: ACN) to obtain a final product NYM032 as a white solid (10.2 mg, 5.64 µmol, purity 95.1%, TFA).

### Example 2 Preparation process of compound ⁶⁸Ga-NYM032

The synthesized NYM032 precursor was further chelated with radioactive isotope gallium [⁶⁸Ga], to obtain a ⁶⁸Ga-NYM032 tracer that can be used for clinical PET/CT tracing. A labeling process is shown below. The entire labeling process can be completed within 20 minutes. Specific steps are as follows: ⁶⁸Ga radionuclide was obtained by eluting a germanium gallium generator with a 0.1M hydrochloric acid solution. 5 mL of the ⁶⁸Ga radionuclide (25 mCi) solution was taken and added to a reaction flask, and then 3 mL to 5 mL of 0.3 mol/L acetic acid/sodium acetate buffer solution was added to the reaction flask. Thus, a volume ratio of ⁶⁸Ga to the buffer solution was 1:1, and the pH value was adjusted to 4.0 to 6.0. An appropriate amount of NYM032 precursor compound was taken and added into sterile water for injection to prepare a 1 mg/mL precursor solution. Then, 49 nmol (100 µg) of the 1 mg/mL precursor solution was taken and added into the reaction flask, the mixture was reacted at 80°C for 6 minutes, and the reaction solution was cooled for 1 minute after the reaction was completed. 5 mL of sterile water for injection was taken by using a 10 mL sterile syringe and added into the reaction flask to dilute the reaction solution to achieve a purpose of lowering a temperature of the reaction solution. Then, all the liquid in the reaction flask was drawn into the syringe, and a pre-activated Sep-Pak C-18 cartridge was taken out and attached to an outlet of the syringe. The reaction dilution was pushed to flow through the Sep-Pak C-18 cartridge, and the target product ⁶⁸Ga-NYM032 was adsorbed by the C-18 cartridge. Then, the C-18 cartridge was rinsed with a 1 mL of 70% medical grade anhydrous ethanol solution. The rinse solution was filtered through a 0.22 µm sterile filter membrane and flowed into a sterile vacuum flask. Then, 5 mL to 8 mL of a saline solution was added into the sterile vacuum flask, to obtain a ⁶⁸Ga-NYM032 sterile injection solution.

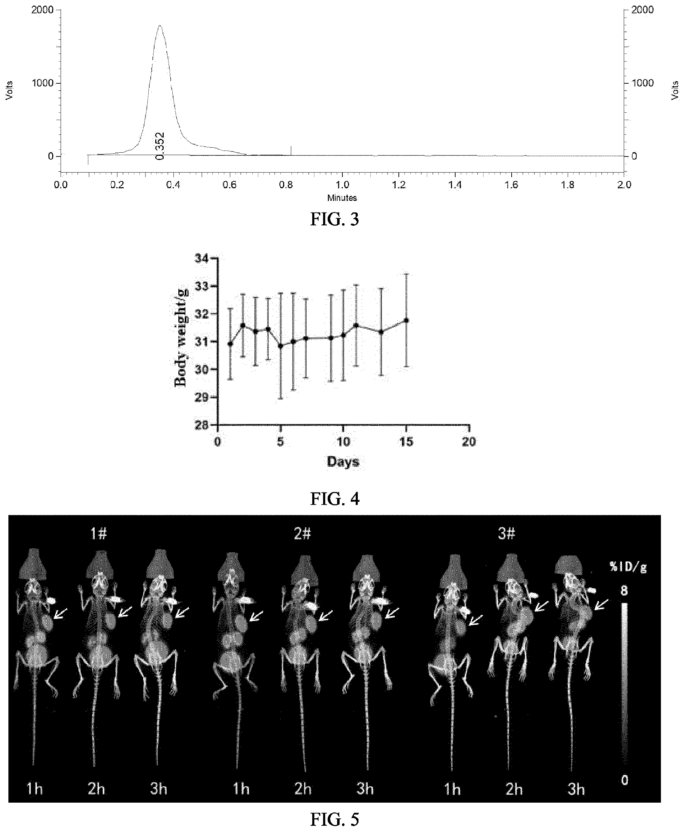

Radioactive thin-layer chromatography was used for quality control of the above product, with a glass fiber paper as the carrier, an 0.5 M citric acid/sodium citrate buffer solution (pH=5) as the developing agent. The glass fiber paper was taken, and the sample was taken with a pipette and gently spotted onto the glass fiber paper at a position 1.5 cm from a bottom. The spotted paper was putted into a test tube with 500 µL of the 0.5M citric acid/sodium citrate buffer (pH=5) pre-added, when the developing agent was spread to a position 2.5 cm from a top of the chromatography paper, the paper was taken out and dried, and detected by using a Radio-TLC thin layer scanner. In the 0.5 M citric acid/sodium citrate buffer (pH=5) system, an Rf value of the product ranges from 0.2 to 0.4. As illustrated in FIG. 3, a radioactive thin-layer chromatography scanning purity analysis result indicated that radiochemical purity of ⁶⁸Ga-NYM032 was 100%.

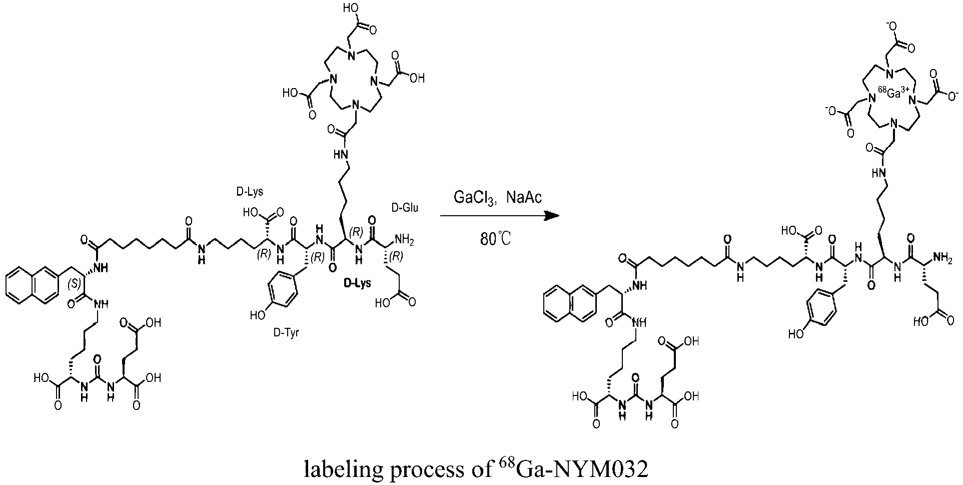

### Example 3 Acute toxicity experiment in ICR mice

ICR mice aged 6 to 8 weeks were purchased from Suzhou Hengjia Biotechnology Co., Ltd. Six mice were randomly selected and each of the six mice was administered with ⁶⁸Ga-NYM032 at a dose ranging from 300 µCi to 400 µCi. General indicators of the animals (animal hair, activity, diet, etc.), mortality of the animals (death time, etc.), and a body weight of the animals were observed 0 days before administration and 2, 4, 6, 8, 10, 12, and 14 days after the administration. The animals were sacrificed and dissected on the last day of the experiment to observe whether there were any abnormalities in their main organs. As illustrated in FIG. 4, during the 14-day observation, none of the mice died and had no abnormal reactions, no abnormalities were observed in their organs during the dissection, and all the mice had gained weight after the experiment. In this way, it is indicated that the ⁶⁸Ga-NYM032 molecule has good safety and can meet the requirements of scientific research and clinical medication.

### Example 4 PET/CT scanning tissue distribution and targeting experiment of ⁶⁸Ga-NYM032 in LNcap model mice

B-NDG mice aged 8 to 9 weeks were purchased from Biocytogen (Beijing) Pharmaceutical Technology Co., Ltd. A B-NDG mouse-based subcutaneous ectopic xenograft tumor model of LNcaP was established. This model is a mouse model constructed with human prostate cancer cells. Three of the animal models were selected, and each of them was given 60 µCi to 80 µCi of the above-mentioned ⁶⁸Ga-NYM032 drug. The animals were pre-anesthetized with an appropriate concentration of isoflurane/air mixed gas before scanning, and then were placed in a MicroPET/CT imaging chamber (model SNPC-303, Super Nova, Pingsheng Healthcare Technology (Kunshan)Inc.), and anesthesia was maintained with isoflurane/air mixed gas. MicroPET/CT scans were respectively carried out 1 hour, 2 hours, and 3 hours after the administration. Scanned images were obtained after reconstruction by device's software and were analyzed using PMOD software. Results are illustrated in FIG. 5, indicating that a biodistribution of the ⁶⁸Ga-NYM032 drug in the LNcaP tumor model mice showed that the ⁶⁸Ga-NYM032 drug was mainly metabolized through their kidneys, with high radioactive accumulation in tumor tissues (indicated by arrows) and relatively low uptake in other tissues.

### Example 5 Estimation of internal irradiation dose of ⁶⁸Ga-NYM032 in human

Four ICR mice were taken, each of the four ICR mice was administered with 100 µCi of radioactive drug ⁶⁸Ga-NYM032, followed by a dynamic scan for 30 minutes. Then, static scans were respectively carried out for 10 minutes at 1 hour, 2 hours, 3 hours, and 4 hours after the administration. Tissue distribution data of ICR mice at different time points were obtained based on scanning data, and then retention time of the radioactive drug ⁶⁸Ga-NYM032 in each organ in the mouse was calculated by using PMOD software. Then, the retention time of the radioactive drug ⁶⁸Ga-NYM032 in each organ in the mouse was extrapolated to that in each organ in human. Then, an internal irradiation dose in human was calculated by using OLINDA software. A calculation result showed that an overall effective dose of human ED was 0.010 mSv/MBq. Calculated according to a clinical administration dose of 111 MBq (3mCi)/person, a total effective radiation dose was 1.221 mSv/person, which was much lower than a dose of 10 mSv to 15 mSv in conventional CT scans of chest and abdomen and was within a safety range. The estimation of the radiation dose is shown in Table 2 below.

**[Table 2]**

| Target organs | ICRP-103 ED; unit: mSv/MBq |
|---|---|
| Adrenal gland | 1.79E-04 |
| Brain | 2.70E-05 |
| Esophagus | 5.31E-04 |
| Eye | 0.00E+00 |
| Gallbladder wall | 1.46E-04 |
| Left colon | 7.19E-04 |
| Small intestine | 1.37E-04 |
| Stomach wall | 1.70E-03 |
| Right colon | 7.19E-04 |
| Rectum | 3.35E-04 |
| Heart wall | 9.61E-05 |
| Kidney | 5.66E-04 |
| Liver | 7.71E-04 |
| Lung | 7.49E-04 |
| Pancreas | 1.38E-04 |
| Prostate | 6.70E-05 |
| Salivary gland | 1.30E-04 |
| Red marrow | 1.32E-03 |
| Osteoblast | 1.03E-04 |
| Spleen | 1.35E-04 |
| Taste | 5.23E-04 |
| Thymus | 1.22E-04 |
| Thyroid | 5.28E-04 |
| Bladder wall | 5.77E-04 |
| whole body | 0.0103 |

### Example 6 Preparation process of compound NYM033

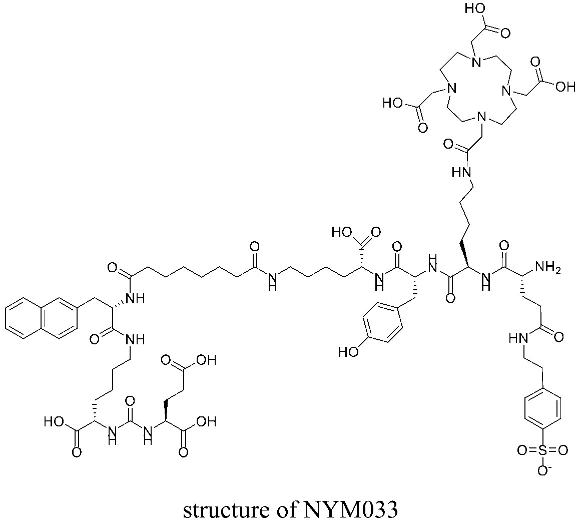

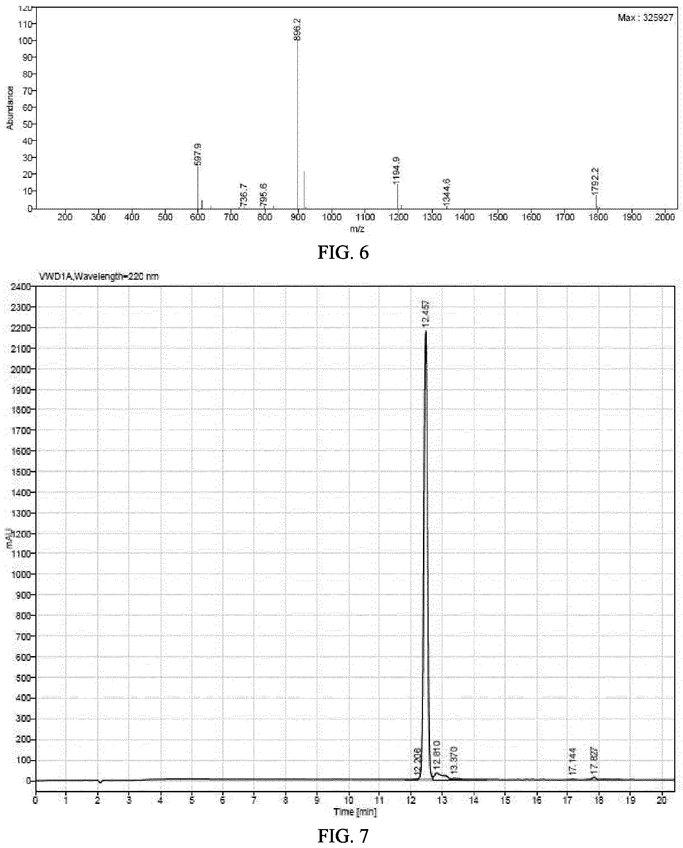

The LC-MS spectrum is shown in FIG. 6, and the HPLC spectrum is shown in FIG. 7.

### Synthesis route:

NYM033 was also obtained by using a similar method to that used for NYM032;

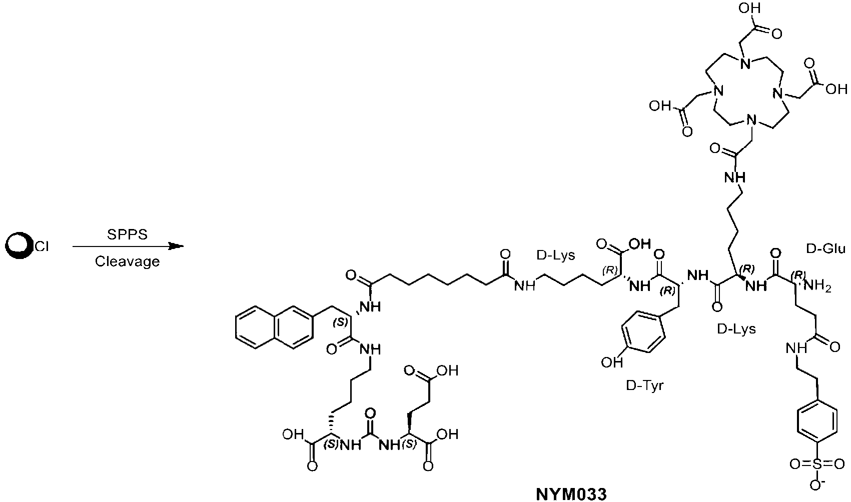

Synthesis of peptide: the peptide was synthesized by using the Fmoc synthesis method.
1. Preparation of resin: 2-CTC (0.30 mmol, 1.00 mmol, Sub 0.50 mmol/g) and Fmoc-Lys(Dde)-OH (0.15 g, 0.30 mmol, 1.00 eq) were added into a solid-phase reactor, DCM (20.0 mL) was added, and the mixture was bubbled with N₂. Then, DIEA (0.15 g, 1.20 mmol, 0.21 mL, 4.00 eq) was added dropwise, the mixture was reacted at 20°C with N₂ bubbling for 2 hours. MeOH (0.60 mL) was added, and the mixture was continuously stirred for 30.0 minutes. Subsequently, a resin was washed with DMF for 5 times, using 50 mL of DMF each time, to obtain a resin.
2. 50 mL of a DMF solution containing 20% piperidine was added into the reactor, the mixture was reacted with N₂ bubbling for 30.0 minutes, and the resin was washed with DMF (50.0 mL*5).
3. Condensation: H-Glu(OtBu)-OtBu.HCl (0.27 g, 0.90 mmol, 3.00 eq), CDI (0.15 g, 0.90 mmol, 3.00 eq), and DMAP (0.12 g, 0.90 mmol, 3.00 eq) were dissolved in THF (20.0 mL). Then, DIEA (0.09 g, 0.60 mmol, 0.12 mL, 2.00 eq) was added dropwise, and the mixture was reacted at 20°C for 1 hour. Then, this solution was added to the resin, and the mixture was stirred at 20°C for 12 hours with N₂ bubbling. Then, the resin was washed with DMF for 5 times, using 50 mL of DMF each time.
4. 3% hydrazine hydroxide (50.0 mL) was added to the resin, and the mixture was continuously reacted with N₂ bubbling for 1 hour. Then, the resin was washed with DMF for 5 times, using 50 mL of DMF each time, and filtered.
5. Condensation: Fmoc-2-Nal-OH (0.21 g, 0.45 mmol, 1.50 eq) and HBTU (0.15 g, 0.42 mmol, 1.43 eq) were dissolved in 20.0 mL DMF, the solution was added to the resin, and the mixture was bubbled with N₂. Then, DIEA(0.12 g, 0.90 mmol, 0.05 mL, 3.00 eq) was added to the mixture, and the mixture was reacted at 20°C with N₂ bubbling for 30.0 minutes. Then, the resin was washed with DMF for 5 times, using 50 mL of DMF each time.
6. A 50 mL of DMF solution containing 20% piperidine was added into the resin, and the mixture was continuously reacted with N₂ bubbling for 30.0 minutes. Then, the resin was washed with DMF for 5 times, using 50 mL of DMF each time, and filtered, to obtain a resin.
7. Steps 5 and 6 above were repeated to sequentially couple amino acid in Table 3 (serial numbers c to h):

**[Table 3]**

| **Serial number** | **Coupling amino acid** | **Coupling reagent** |
|---|---|---|
| a | Fmoc-Lys(Dde)-OH (1.00 *eq*) | DIEA (4.00 *eq*) |
| b | H-Glu(OtBu)-OtBu.HCl (3.00 *eq*) | CDI (3.00 eq), DMAP (3.00eq), DIEA (2.00 *eq*) |
| c | Fmoc-2-Nal-OH (1.50 *eq*) | HOAT (1.43 eq), DIC (1.50 *eq*) |
| d | Compound (6) (1.50 *eq*) | DIEA (3.00 *eq*) |
| e | Fmoc-D-Tyr(tBu)-OH (3.00 *eq*) | HOAT (2.85 eq), DIC (3.00 *eq*) |
| f | Dde-D-Lys(Fmoc)-OH (1.50 *eq*) | HOAT (1.43 eq, DIC (1.50 *eq*) |
| g | DOTA-tri(t-butyl ester) (2.00 *eq*) | HBTU (1.90 eq), DIEA (4.00 *eq*) |
| h | Fmoc-D-Glu(OAll)-OH (1.50 *eq*) | HOAT (1.43 eq), DIC (1.50 *eq*) |
| i | 4-(2-aminoethyl)benzenesulfonic acid hydrochloride (2.00 *eq*) | DIEA (4.00 *eq*) |

8. Deprotection: PhSiH (10.0 eq) and Pd (PPh₃)₄ (0.10 eq) were dissolved in DCM (20.0 mL) solvent and added to the above resin. The mixture was reacted with N₂ bubbling for 1 hour, and then the resin was washed with DMF for 5 times, using 50 mL of DMF each time, and filtered.
9. Condensation: pentafluorophenol was dissolved in DCM (20.0 mL) solvent, the solution was added to the resin, and the mixture was reacted with N₂ bubbling. Then, DIC (0.08 g, 0.60 mmol, 0.09 mL, 2.00 eq) was added, and the mixture was reacted at 20°C with N₂ bubbling for 2 hours. Then, the resin was washed with DMF for 5 times, using 50 mL of DMF each time.
10. Condensation: 4-(2-aminoethyl)benzenesulfonic acid hydrochloride (0.14 g, 0.60 mmol, 2.00 eq) was taken and dissolved in DMF (20.0 mL), the solution was added to the resin, and the mixture was bubbled with N₂. Then, DIEA (0.15 g, 1.20 mmol, 0.21 mL, 4.00 eq) was added, and the mixture was reacted at 20°C with N₂ bubbling for 30 minutes. Then, the resin was washed with DMF for 5 times, using 50 mL of DMF each time.
11. A DMF solution containing 20% piperidine was added to the resin, and the mixture was continuously stirred for 30.0 minutes to remove an Fmoc protecting group. The deprotection reaction was monitored with a ninhydrin detection reagent, and the resin was washed five times with DMF after the reaction was completed.
12. Cleavage and purification of peptide: a peptide with a side-chain protecting group and a cleavage solution (90% TFA, 2.5% H₂O, 2.5% 3-Mercaptopropionic acid, 5% TIS) were added into a flask and stirred at room temperature for 2 hours for deprotection. The mixture was precipitated with tert-butyl methyl ether and placed at -20°C for 5.0 minutes, and filtered to collect a filter cake. Then, the filter cake was washed with tert-butyl methyl ether more than twice and was vacuum dried for 2 hours. The crude product was purified by using preparative high-performance liquid chromatography (A: an aqueous solution of 0.075% TFA, B: ACN). The crude product was purified again by using the preparative high-performance liquid chromatography (condition: the TFA solution was changed into a NaHCO₃ solution), to obtain a final product NYM033 as a white solid (0.1 mg, 5.31 µmol, purity 95.2%).

### Example 7 Preparation process of compound ⁶⁸Ga-NYM033

The synthesized NYM033 precursor was further chelated with radioactive isotope gallium [⁶⁸Ga], to obtain a ⁶⁸Ga-NYM033 tracer that can be used for clinical PET/CT tracing. A labeling technology of the ⁶⁸Ga-NYM033 tracer is well-established. A labeling process is shown below. The entire labeling process can be completed within 20 minutes. Specific steps are as follows. ⁶⁸Ga radionuclide was obtained by eluting a germanium gallium generator with a 0.1M hydrochloric acid solution. 5 mL of the ⁶⁸Ga radionuclide (25 mCi) solution was taken and added to a reaction flask, and then 3 mL to 5 mL of a 0.3 mol/L acetic acid/sodium acetate buffer solution was added to the reaction flask. Thus, a volume ratio of ⁶⁸Ga to the buffer solution was 1:1, and the pH value was adjusted to 4.0 to 6.0. An appropriate amount of NYM033 precursor compound was taken and added into sterile water for injection to prepare a 1 mg/mL precursor solution. Then, 49 nmol (100 µg) of the 1 mg/mL precursor solution was taken and added into the reaction flask, the mixture was reacted at 80°C for 6 minutes, and the reaction solution was cooled for 1 minute after the reaction was completed. 5 mL of sterile water for injection was taken by using a 10 mL sterile syringe and added into the reaction flask to dilute the reaction solution to achieve a purpose of lowering a temperature of the reaction solution. Then, all the liquid in the reaction flask was drawn into the syringe, and a pre-activated Sep-Pak C-18 cartridge was taken out and attached to an outlet of the syringe. The reaction dilution was pushed to flow through the Sep-Pak C-18 cartridge, and the target product ⁶⁸Ga-NYM033 was adsorbed by the C-18 cartridge. Then, the C-18 cartridge was rinsed with a 1 mL of 70% medical grade anhydrous ethanol solution. The rinse solution was filtered through a 0.22 µm sterile filter membrane and flowed into a sterile vacuum flask. Then, 5 mL to 8 mL of a saline solution was added into the sterile vacuum flask, to obtain a ⁶⁸Ga-NYM032 sterile injection solution.

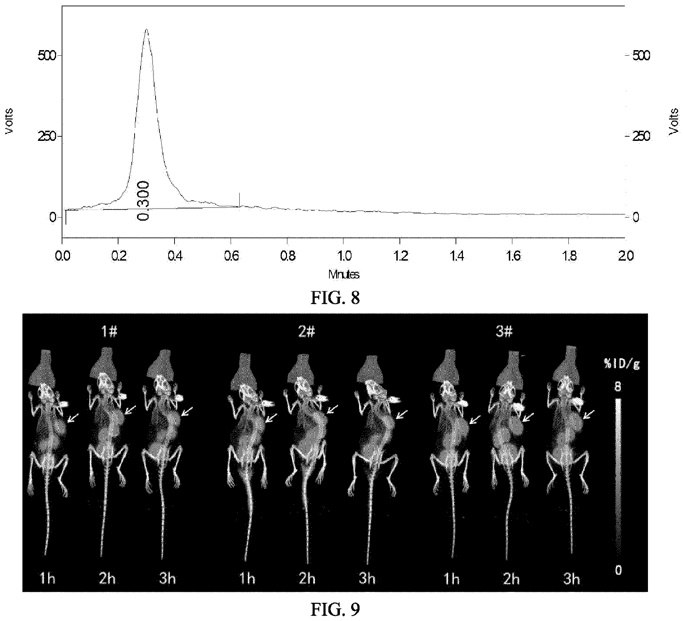

Radioactive thin-layer chromatography was used for quality control of the above product, with a glass fiber paper as the carrier, an 0.5 M citric acid/sodium citrate buffer solution (pH=5) as the developing agent. The glass fiber paper was taken, and the sample was taken with a pipette and gently spotted onto the glass fiber paper at a position 1.5 cm from a bottom. The spotted paper was putted into a test tube with 500 µL of the 0.5M citric acid/sodium citrate buffer (pH=5) pre-added, when the developing agent was spread to a position 2.5 cm from a top of the chromatography paper, the paper was taken out and dried, and detected by using a Radio-TLC thin layer scanner. In the 0.5 M citric acid/sodium citrate buffer (pH=5) system, an Rf value of the product ranges from 0.2 to 0.4. As illustrated in FIG. 8, a radioactive thin-layer chromatography scanning purity analysis result indicated that radiochemical purity of ⁶⁸Ga-NYM033 was 100%.

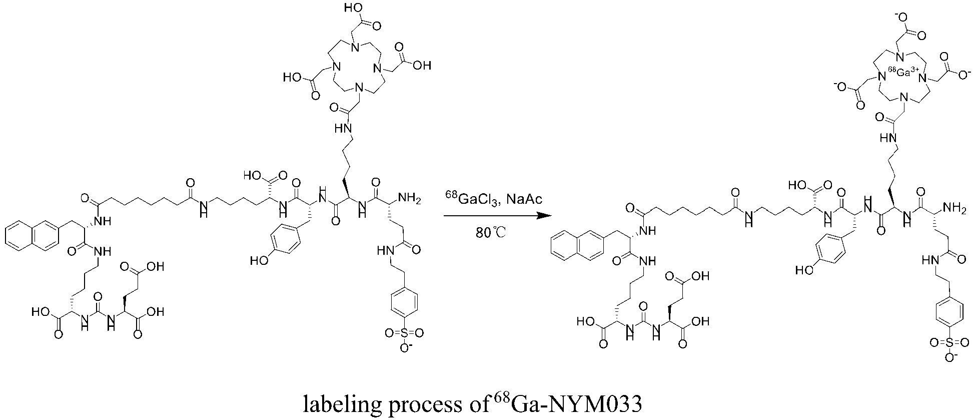

### Example 8 PET/CT scanning tissue distribution and targeting experiment of ⁶⁸Ga-NYM033 in LNcap model mice

B-NDG mice aged 8 to 9 weeks were purchased from Biocytogen (Beijing) Pharmaceutical Technology Co., Ltd. A B-NDG mouse-based subcutaneous ectopic xenograft tumor model of LNcaP was established. This model is a mouse model constructed with human prostate cancer cells. Three of the animal models were selected, and each of them was given 60 µCi to 80 µCi of the above-mentioned ⁶⁸Ga-NYM033 drug. The animals were pre-anesthetized with an appropriate concentration of isoflurane/air mixed gas before scanning, and then were placed in a MicroPET/CT imaging chamber (model SNPC-303, Super Nova, Pingsheng Healthcare Technology (Kunshan)Inc.), and anesthesia was maintained with isoflurane/air mixed gas. MicroPET/CT scans were respectively carried out 1 hour, 2 hours, and 3 hours after the administration. Scanned images were obtained after reconstruction by device software and were analyzed using PMOD software. Results are illustrated in FIG. 9, indicating that a biodistribution of the ⁶⁸Ga-NYM033 drug in the LNcaP tumor model mice showed that the ⁶⁸Ga-NYM033 drug was mainly excreted through their kidneys, with high radioactive accumulation observed in tumor tissues (indicated by arrows) and relatively low uptake in other tissues.

### Example 9 Competitive inhibition experiment of ⁶⁸Ga-NYM032 and ⁶⁸Ga-NYM033 in LNcaP tumor-bearing mouse model

B-NDG mice aged 8 to 9 weeks were purchased from Biocytogen (Beijing) Pharmaceutical Technology Co., Ltd. A B-NDG mouse-based subcutaneous ectopic xenograft tumor model of LNcaP was established. This model is a mouse model constructed with human prostate cancer cells.

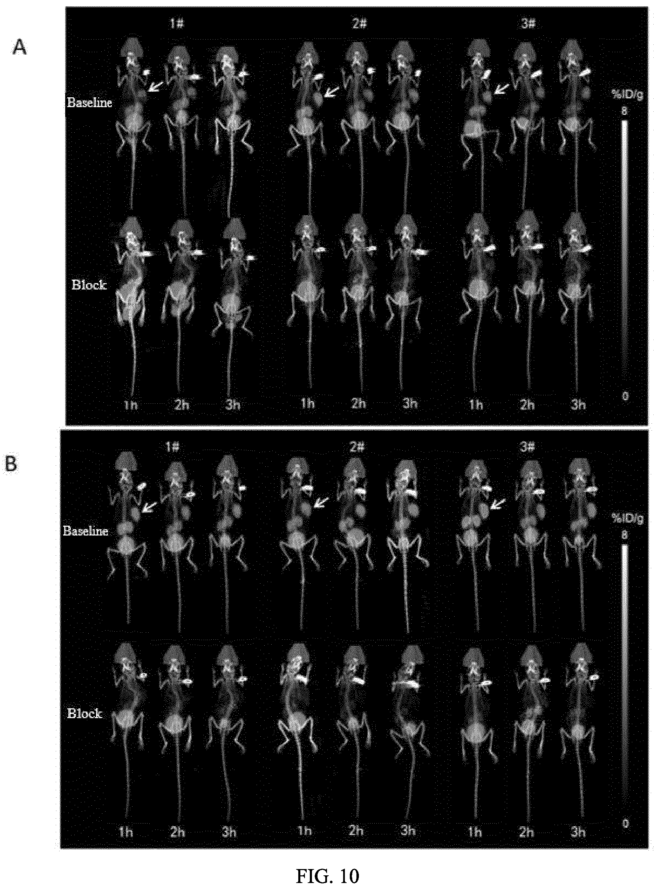

The experiment was conducted in two parts: a baseline experiment and a blocking experiment. The baseline experiment was divided into two groups. Three of the above-mentioned model mice were randomly selected for each of the two groups. For one of the two groups, the mice were injected with 200 µL of approximately 40 µCi of ⁶⁸Ga-NYM032 via their tail veins. For another one of the two groups, the mice were injected with 200 µL of approximately 40 µCi of ⁶⁸Ga-NYM033 via their tail veins. MicroPET/CT scans were respectively carried out at 1 hour, 2 hours, and 3 hours after the injection of ⁶⁸Ga-NYM032 and ⁶⁸Ga-NYM033. Scanned images were obtained after reconstruction by device software and analyzed by using PMOD software. The blocking experiment was divided into two groups, with the same grouping and animals as in the baseline experiment. For one of the two groups, the mice were injected with cold drug NYM032 via their tail veins at an injection dose 40 times the mass of the hot drug (⁶⁸Ga-NYM032) to be injected. For another one of the two groups, the mice were injected with cold drug NYM033 via their tail veins at an injection dose 40 times the mass of hot drug (⁶⁸Ga-NYM033) to be injected. 30 minutes after the cold drug was administered, the corresponding groups were injected with hot drugs ⁶⁸Ga-NYM032 and ⁶⁸Ga-NYM033, respectively. MicroPET/CT scans were respectively carried out at 1 hour, 2 hours, and 3 hours after the hot drug was administered. Scanned images were obtained after reconstruction by the device's software and analyzed by using the PMOD software. The images as shown in FIG. 10 indicate that the addition of the cold drugs can significantly block the uptake of ⁶⁸Ga-NYM032 and ⁶⁸Ga-NYM033 in the tumor (indicated by arrows), thereby confirming that the uptake of ⁶⁸Ga-NYM032 and ⁶⁸Ga-NYM033 by the tumor was specific targeted.

### Example 10 PET/CT imaging of ⁶⁸Ga-NYM032 and ⁶⁸Ga-NYM033 molecules in clinical research trial

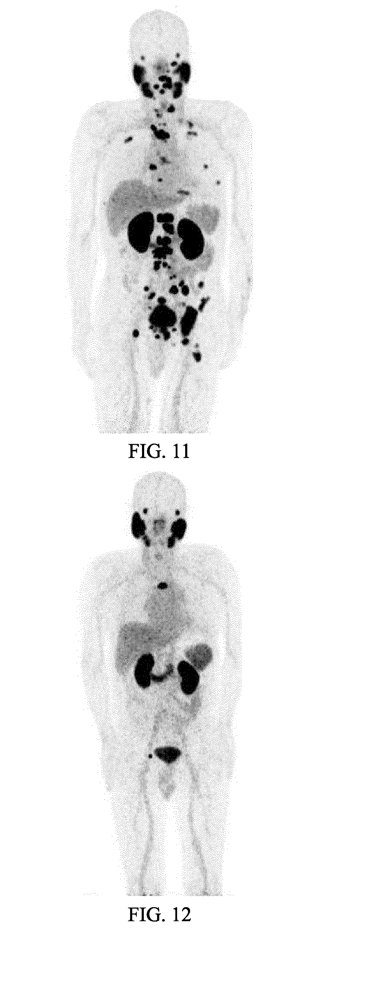

(1) Brief information of the patient: patient 1 was admitted to the hospital due to "increased nocturia for one year and intermittent painless hematuria for four months". The patient's blood test showed abnormally elevated prostate tumor indicators, CT and B-ultrasound showed prostatic hyperplasia with calcification, and suspected thickening of a posterior bladder wall. The patient was then enrolled in a clinical research trial of a PSMA tracer, and whole-body PET/CT tomographic imaging was performed at 1 hour after injection of 3.31 mCi of ⁶⁸Ga-NYM032. Examination results are shown in FIG. 11, in which for Patient 1, multiple foci of abnormally increased radioactive uptake were found in a skull, multiple sites in a spine, a sternum, multiple sites in bilateral ribs, a left scapula, multiple sites in a pelvis, an upper section of a left femur, and a lower section of a left tibia. The maximum standardized uptake value (SUVmax) of 31.7. The CT images of the corresponding sites showed (osteoblastic) bone destruction, which was consistent with prostate cancer with systemic multiple bone metastases. There were multiple slightly enlarged lymph nodes with increased expression of PSMA receptors in a left clavicular region, mediastinum, retroperitoneum, and bilateral iliac vessels, which was considered to be caused by the metastasis. It is indicated that the tumor and its metastatic lesion can radioactively take up the ⁶⁸Ga-NYM032 drug in a short period of time, leading to a good imaging effect that can detect a small tumor and its metastatic foci with clear imaging interface.
(2) Brief information of the patient: patient 2 had undergone multimoda treatment for prostate cancer, MT, for more than two years. A recent examination showed Patient 2's Total Prostate-Specific Antigen, TPSA, level of 1.38ng/ml, and he had no other obvious discomforts. In order to evaluate his overall systemic condition, he was enrolled in a clinical research trial of a PSMA tracer, and whole-body PET/CT tomographic imaging was performed at 1 hour after injection of 3.30 mCi of ⁶⁸Ga-NYM033. Examination results are shown in FIG. 12, in which after the comprehensive treatment for prostate cancer in Patient 2, expression of PSMA receptors in T4 and right acetabulum was significantly abnormally increased, with an SUVmax of 21.7, and CT images of the corresponding sites showed no obvious bone destruction, which was considered to be caused by metastasis. A right anterior chest wall was slightly thickened with local nodularity and slightly increased radioactive uptake, with an SUVmax of 3, which was considered to be an inflammatory change. It is indicated that the tumor and its metastatic lesion can radioactively take up the ⁶⁸Ga-NYM033 drug in a short period of time, leading to a good imaging effect that can detect a small tumor and its metastatic foci with clear imaging interface.

### Example 11 PET/CT Imaging of ⁶⁸Ga-NYM032 and ⁶⁸Ga-PSMA-11 in clinical research trial

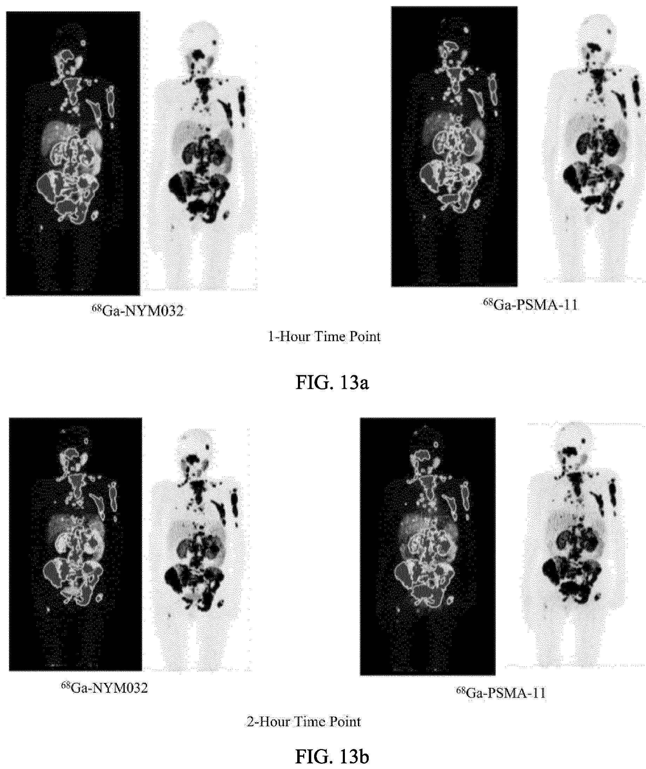

Brief information of the patient: patient 3 was diagnosed with prostate cancer and underwent radical prostatectomy and endocrine therapy. Whole-body bone scintigraphy revealed systemic multiple bone metastases. Whole-body PET/CT tomographic imaging was performed at 1 hour and 2 hours after injection of 2.8 mCi of ⁶⁸Ga-NYM032. Two days after the injection of ⁶⁸Ga-NYM032, the patient was injected with 2.8 mCi of ⁶⁸Ga-PSMA-11 and whole-body PET/CT tomographic imaging was performed at 1 hour and 2 hours after the injection. Imaging results are shown in FIGS. 13a and 13b, in which Patient 3 had multiple metastatic bone lesions throughout the body, which was consistent with prostate cancer with systemic multiple bone metastases. There was no significant difference in radioactive uptake of ⁶⁸Ga-NYM032 at the imaging sites of tumors and tumor metastases compared with that of currently marketed PSMA diagnostic product ⁶⁸Ga-PSMA-11. Moreover, the uptake of ⁶⁸Ga-NYM032 by kidney was lower than that of ⁶⁸Ga-PSMA-11, resulting in lower radioactive exposure in the body. It is indicated that ⁶⁸Ga-NYM032 can undergo radioactive uptake in a short period of time, leading to a good imaging effect that can detect a small tumor and its metastatic foci with clear imaging interface.

### Example 12 PET/CT Imaging of ⁶⁸Ga-NYM032 and ⁶⁸Ga-PSMA-617 in clinical research trial

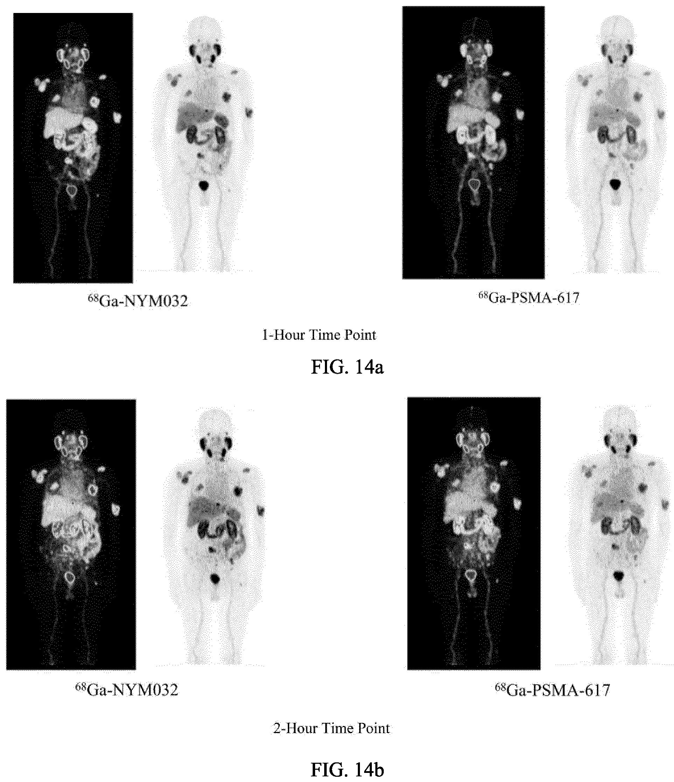

Brief information of the patient: patient 4 was diagnosed with prostate cancer and subsequently underwent laparoscopic radical prostatectomy. A bone scan at the same time showed increased metabolism in a sixth right rib. After surgery, Patient 4 was treated with Zoladex, Casodex, and Abiraterone. A bone scan indicated a lesion in a sixth right anterior rib, and newly added bone lesions in a seventh right posterior rib, an eighth right posterior costovertebral joint, left scapula, and a middle section of a left humerus. Then, the patient was initiated treatment with Denosumab and was treated with Polaprezinc for 10 months. a bone scan imaging performed subsequently showed that compared with a previous whole-body bone scan, there were multiple metastatic bone lesions throughout the body and the number of lesions increased. After that, the patient taken Chinese traditional medicine, and a PSA level was increased slowly and continuously during the follow-up. Whole-body PET/CT tomographic imaging was performed at 1 hour and 2 hours after injection of 2.8 mCi of ⁶⁸Ga-NYM032. Two days after the injection of ⁶⁸Ga-NYM032, the patient was injected with 2.8 mCi of ⁶⁸Ga-PSMA-617, and whole-body PET/CT tomographic imaging was performed at 1 hour and 2 hours after the injection. Examination results are shown in FIGS. 14a and 14b, patient 4 had multiple metastatic bone lesions throughout the body, which was consistent with prostate cancer with systemic multiple bone metastases throughout the body. Compared with ⁶⁸Ga-PSMA-617, radioactive uptake of ⁶⁸Ga-NYM032 in the tumor was higher, and more metastatic lesions could be found. Meanwhile, the uptake of ⁶⁸Ga-NYM032 was lower in a circulatory system such as a heart and major blood vessels, leading to a lower uptake background, higher tumor uptake, and a higher target-to-background ratio. Further, the tumor imaging was more obvious and clearer, making it more valuable for clinical applications.

### Example 13 preparation process of compound ¹⁷⁷Lu-NYM032 molecule

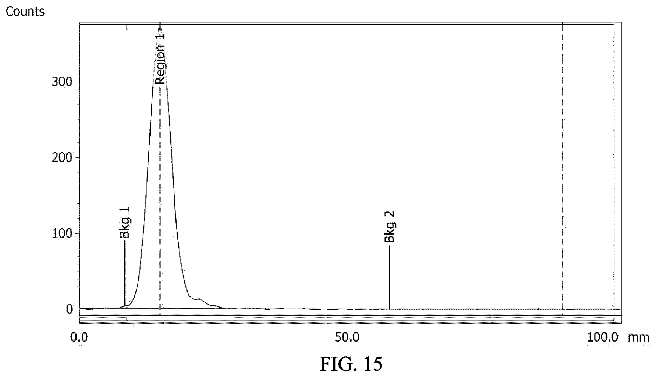

40 to 60 mCi of lutetium [¹⁷⁷Lu]-lutetium chloride solution was transferred to a 10 mL sterile reaction flask, and the solution was neutralized to a pH value ranging from 4 to 4.5 by using a sodium acetate buffer solution (0.15 mol/L). The synthesized NYM032 precursor solution was completely transferred to the reaction flask, mixed and placed in a heater for reaction at 80°C for 12 minutes. A disposable Sep-Pak C-18 cartridge was taken and pre-activated by using10 mL of anhydrous ethanol and 20 mL of sterile water for injection. 10 mL of sterile syringe was taken, 5 mL of sterile water for injection was pre-aspirated, and then all the reaction liquid was aspirated. Then, the C-18 cartridge was connected to push the liquid in the syringe to flow through the C-18 cartridge, and the target product ¹⁷⁷Lu-NYM032 was adsorbed on the C-18 cartridge. Then, 20 mL of sterile water for injection was aspirated to rinse the C-18 cartridge. Finally, the C-18 cartridge was rinsed by using 1.2 mL of 70% anhydrous ethanol solution, and the target product ¹⁷⁷Lu-NYM032 was eluted into a transfer bottle. A syringe pre-aspirated with 3.8 mL of saline was used to inject the saline into the transfer bottle and mix evenly. All the product solution (crude product) in the transfer bottle was aspirated and filtered through a 0.22 µm sterile filter membrane into a 10 mL closed sterile vial to obtain a sterile injection solution of ¹⁷⁷Lu-NYM032. A duration required for the synthesis preparation was about 40 minutes, and a radiochemical purity of the product was greater than 95% as determined by Radio-iTLC method, as shown in FIG. 15.

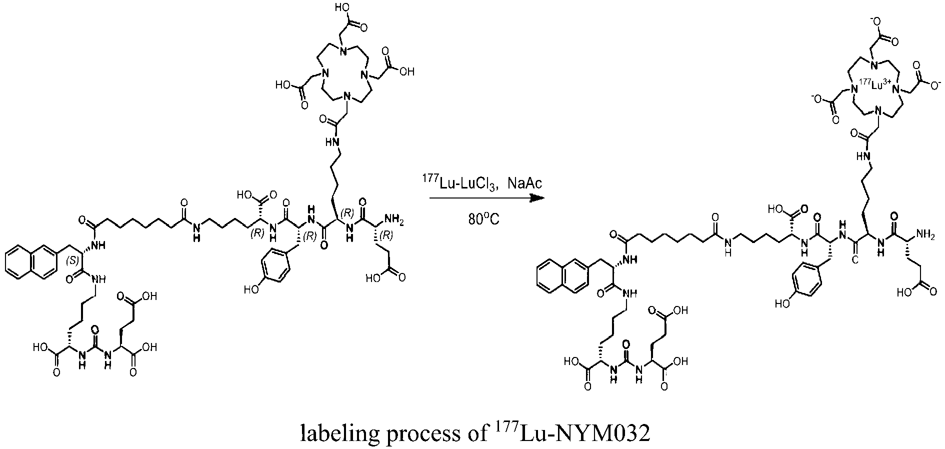

### Example 14 Efficacy experiment of ¹⁷⁷Lu-NYM032 in LNcaP tumor model

Experimental animals were purchased from Biocytogen (Beijing) Pharmaceutical Technology Co., Ltd. A B-NDG mouse-based subcutaneous ectopic xenograft tumor model of LNcaP was established. This model was a mouse model constructed with human prostate cancer cells.

Twelve mice were randomly selected for the experiment. They were divided into two groups including a treatment group and a control group, with 3 tumor mice in each group. For the treatment group, each animal was injected with 100 µL of saline solution of ¹⁷⁷Lu-NYM032 via its tail vein, at a dosage of 1 mCi. For the control group, each animal was injected with 100 µL of saline solution via its tail vein. A body weight and a tumor volume of all the experimental mice were recorded on the day of the injection. The tumor volume (long diameter and short diameter) was measured and the body weight was weighed every 3 days. Meanwhile, a state of the mice was observed and an accurate record was made.

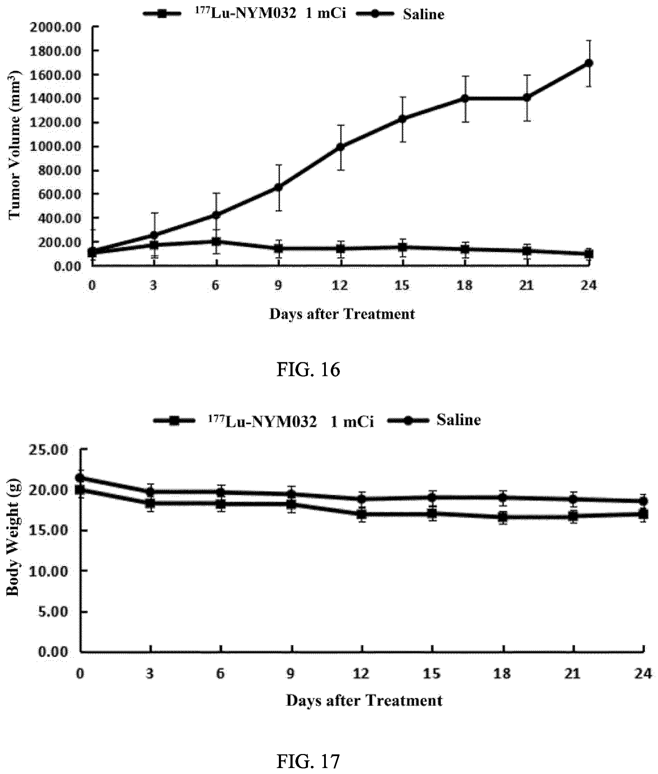

The long diameter and the short diameter of the tumor measured during tumor efficacy evaluation were used to calculate the tumor volume. A calculation formula was as follows: the tumor volume (TV) = a×b²/2 ("a" represents the long diameter and "b" represents the short diameter). A trend graph of the tumor volume in each group was shown in FIG. 16, and a trend graph of a change in the body weight of the mice was shown in FIG. 17. It can be seen that ¹⁷⁷Lu-NYM032 inhibits tumor growth more significantly than saline, and there is no obvious change in the body weight of the mice, indicating that ¹⁷⁷Lu-NYM032 had a good anti-tumor effect and relatively good safety.

### Example 15 SPECT/CT Imaging of ¹⁷⁷Lu-NYM032 in clinical research trial

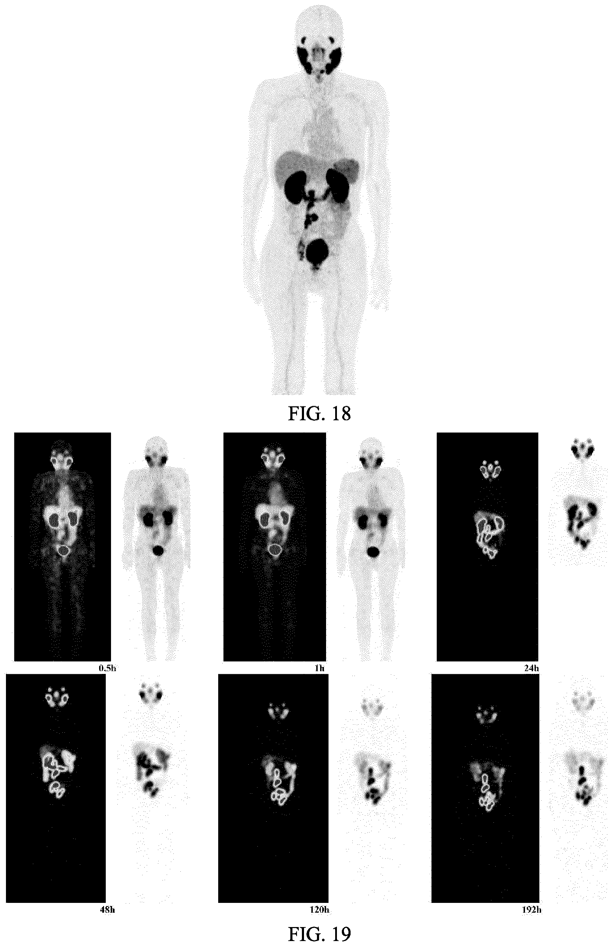

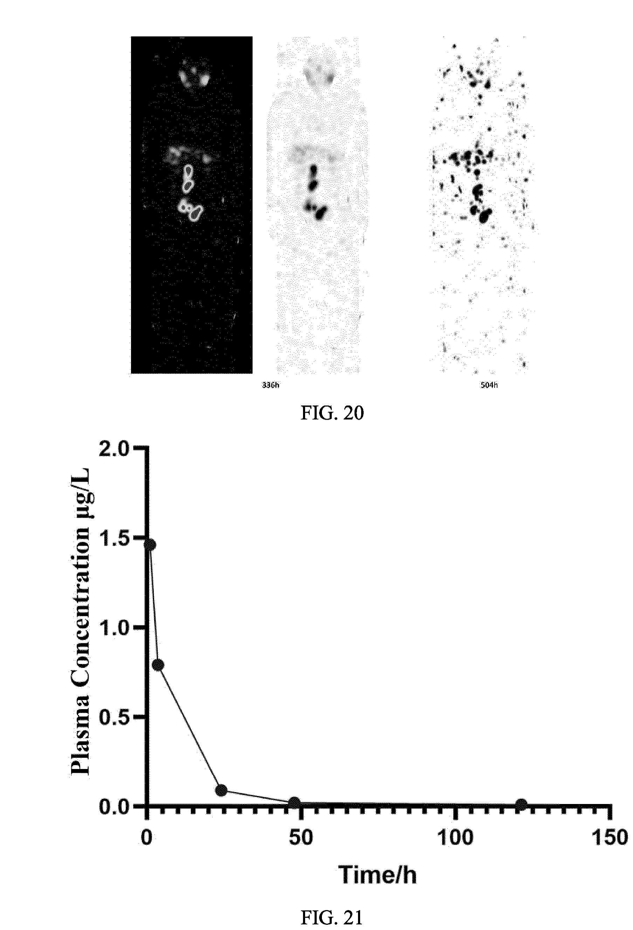

Brief information of patient: patient 5 was a 72-year-old male weighing 56 kg. He underwent a prostate puncture under the guidance of B-ultrasound, and postoperative pathology indicated prostate cancer. Subsequently, androgen suppression therapy and chemotherapy were carried out. The patient was enrolled in a clinical research trial of a PSMA tracer. The whole-body PET/CT tomographic imaging was performed one hour after injection of 3.75 mCi of ⁶⁸Ga-NYM032 drug into the patient. Examination results are shown in FIG. 18. It is indicated that patient 5 had multiple metastatic bone lesions throughout the body, which was consistent with prostate cancer with systemic multiple bone metastases. 24 hours after the injection of the ⁶⁸Ga-NYM032 drug, the patient was injected with 18.54mCi of ¹⁷⁷Lu-NYM032 and whole-body SPECT/CT tomographic imaging was performed at 0.5 hours, 1 hour, 24 hours, 48 hours, 120 hours, 192 hours, 336 hours, and 504 hours. Meanwhile, venous blood samples were collected from the patients to measure gamma counts before the administration and 1 hour, 4 hours, 24 hours, 48 hours, and 120 hours after the administration, to calculate a plasma concentration at different time points. Imaging results are shown in FIGS. 19 and 20, indicating that high radioactive uptake could still be observed in a tumor and a lesion area at 192 hours after the injection of ¹⁷⁷Lu-NYM032, an SUVmax was not decreased compared with that at 48 hours after the injection of ¹⁷⁷Lu-NYM032, and an interface was clear; there was still uptake in the lesion at 336 hours and 504 hours after the injection of ¹⁷⁷Lu-NYM032. As shown in FIGS. 19 and 21, ¹⁷⁷Lu-NYM032 in a heart and large blood vessels was basically cleared within 24 hours. Compared with commercially available PSMA-targeted therapeutic product ¹⁷⁷Lu-PSMA-617, ¹⁷⁷Lu-NYM032 had a shorter half-life in a circulatory system. Meanwhile,¹⁷⁷Lu-NYM032 had a longer tumor retention duration, higher tumor uptake, and higher cumulative tumor dose. In this way, a single-dose administration and/or an administration frequency was/were reduced. As a result, toxicity to healthy organs and tissues was reduced.

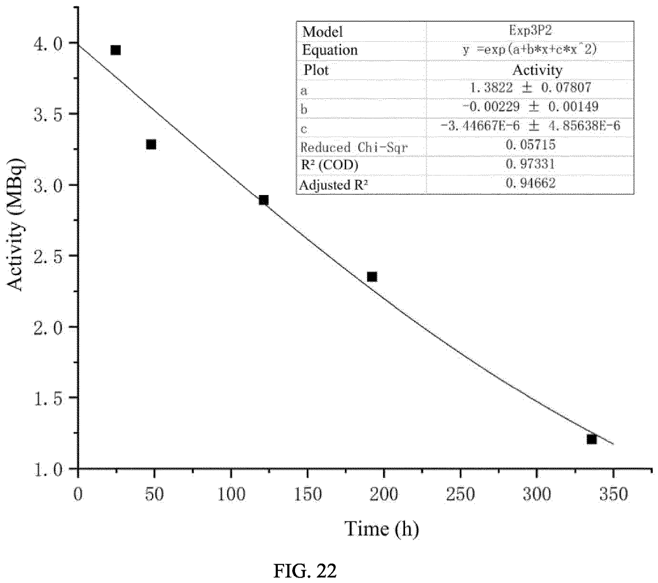

Tissue distribution data of ¹⁷⁷Lu-NYM032 in the body at the different time points could be obtained based on the scanning data, and irradiation dose of all the lesions in the body at the different time points could be calculated by using PMOD software. A relationship between radioactive activity in lesions in vivo and time is shown in FIG. 22.

### Example 16. SPR affinity detection of NYM032 molecule

An affinity detection was carried out by using SPR: PSMA protein (purchased from ACROBiosystems, a concentration of 0.325 mg/mL) was immobilized on a CM5 chip with Acetate buffer (a pH value of 5.0, Cytiva), the affinity detection was performed by using a Biacore 8K protein interaction system, and the running buffer was Tris-P+ (50 mM Tris, 150 mM NaCl, and 0.05% (v/v) P20 (Tween 20)). DMSO solutions of the NYM032 molecules at different concentrations were prepared as mobile phases to detect a binding ability of the NYM032 molecule to the PSMA protein. The binding strength between the NYM032 molecule and the PSMA protein was represented by an equilibrium dissociation constant K_{D} (K_{d}/Kₐ) value, where K_{d} represents a dissociation constant and Ka represents a binding constant. The smaller the K_{D} value, the higher the affinity between the compound and the protein. Experimental results showed that the equilibrium dissociation constant K_{D} value between the NYM032 molecule and the PSMA protein was 0.318±0.046nM. The equilibrium dissociation constant K_{D} value between the NYM032 molecule and the PSMA protein was lower than a nanomolar level, indicating that the NYM032 molecule had a relatively strong affinity for the PSMA protein.

The description of the reference to the terms such as "an embodiment," "some embodiments," "an example," "a specific example," or "some examples," throughout this specification means that a particular feature, structure, material, or characteristic described in connection with the embodiment or example is included in at least one embodiment or example of the present disclosure. In this specification, exemplary descriptions of the foregoing terms are not necessarily referring to the same embodiment or example of the present disclosure. Furthermore, the particular features, structures, materials, or characteristics may be combined in any suitable manner in any one or more embodiments or examples. In addition, different embodiments or examples and features of different embodiments or examples described in the specification may be combined by those skilled in the art, unless they are contradictory to each other.

Although embodiments of the present disclosure have been shown and described above, it can be understood that the above embodiments are illustrative and should not be construed as limitations of the present disclosure. Those skilled in the art can make changes, modifications, alternatives, and variations to the above embodiments without departing from scope of the present disclosure.

## Claims

1. A compound, or a pharmaceutically acceptable salt, ester or solvate thereof, the compound having a structure represented by Formula (I): 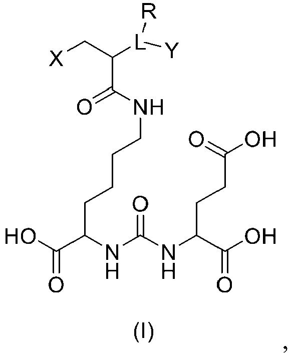 wherein:
L is selected from a polyethylene glycol chain, a hydrophilic amino acid chain, or a carbon chain;
X is selected from a six- to twelve-membered aryl or a five- to twelve-membered heteroaryl, wherein the six- to twelve-membered aryl or the five- to twelve-membered heteroaryl is optionally substituted with one, two, or three substituents selected from OH, halogen, or C₁₋₆ alkyl; and
R and Y are respectively and independently selected from H, an optionally substituted amino acid, or a chelating agent, and at least one of R and Y is the chelating agent.

2. A compound, or a pharmaceutically acceptable salt, ester or solvate thereof, the compound having a structure represented by Formula (II): 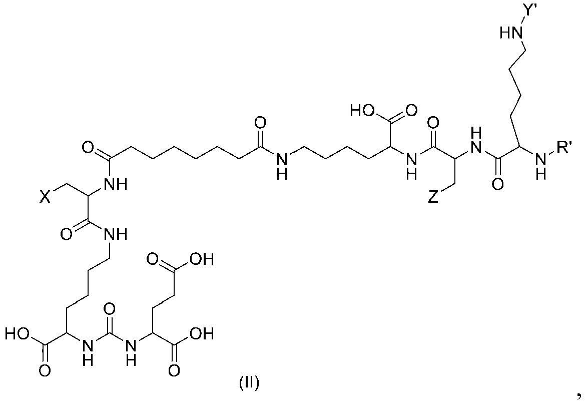
wherein:
X is selected from a six- to twelve-membered aryl or a five- to twelve-membered heteroaryl, wherein the six- to twelve-membered aryl or five- to twelve-membered heteroaryl is optionally substituted with one, two, or three substituents of OH, halogen, or C₁₋₆ alkyl;
Z is selected from 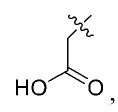 -COOH, -OH, C₁₋₆ alkyl, and six- to twelve-membered aryl, wherein the C₁₋₆ alkyl or the six- to twelve-membered aryl is optionally substituted with one, two, or three substituents of OH, halogen, or C₁₋₆ alkyl; and
R' and Y' are each independently selected from H, an optionally substituted amino acid, or a chelating agent, and at least one of R' and Y' is the chelating agent.

3. The compound or the pharmaceutically acceptable salt, ester or solvate thereof according to claim 2, wherein:
X is selected from 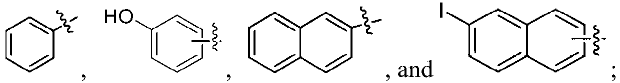
optionally, Z is selected from 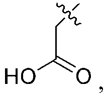 -COOH, -OH, -CH(CH₃)OH, 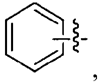 and 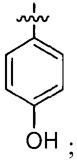 and
optionally, the chelating agent is selected from 1,4,7,10-tetraazacyclododecane-N,N',N",N‴,-tetraacetic acid, 1,4,7-triazacyclononane-1,4,7-triacetic acid, 2-(4,7-bis(carboxymethyl)-1,4,7-triazanonan-1-yl)pentanedioic acid, 2-(4,7,10-tris(carboxymethyl)-1,4,7,10-tetraazacyclododecan-1-yl)pentanedioic acid, 1,4,7-triazacyclononane phosphinic acid, 1,4,7-triazacyclononane-1-[methyl(2-carboxyethyl)phosphinic acid]-4,7-bis[methyl(2-carboxymethyl)phosphinic acid], N'-{5-[acetyl(hydroxy)amino]pentyl}-N-[5-({4-[5-aminopentyl)(hydroxy)amino]-4-oxobutanoyl}amino)pentyl]-N-hydroxysuccinamide, diethylenetriaminepentaacetic acid, trans-cyclohexyl-diethylenetriaminepentaacetic acid, p-isothiocyanatobenzyl-diethylenetriaminepentaacetic acid, 1-(isothiocyanatobenzyl)-3-methyl-diethylenetriaminepentaacetic acid, 1-(isothiocyanatobenzyl)-4-methyl-diethylenetriaminepentaacetic acid, 1-(2)-methyl-4-isothiocyanatobenzyl-diethylenetriaminepentaacetic acid, 1-oxa-4,7,10-triazacyclododecane-4,7,10-triacetic acid, 6-hydrazinonicotinic acid succinimidyl ester hydrochloride, mercaptoacetyltriglycine, or [(R)-2-amino-3-(4-isothiocyanatophenyl)propyl]-trans-(S,S)-cyclohexane-1,2-diaminopentaacetic acid.

4. A complex, the complex being formed by complexing the compound according to claim 1 to 3, or the pharmaceutically acceptable salt, ester, or solvate thereof with a radionuclide A' or a non-radioactive element A', and the complex having a structure represented by Formula (III): 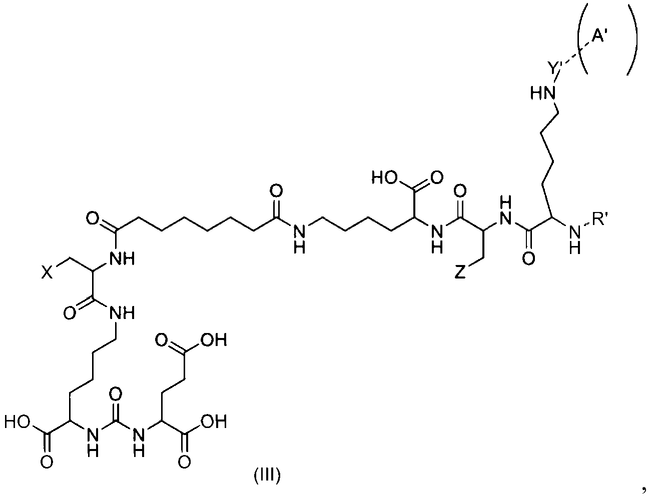 wherein:
Y' is a chelating agent; and
R' is H, an optionally substituted amino acid, or a chelating agent.

5. A complex, the complex being formed by complexing the compound according to any one of claims 1 to 3, or the pharmaceutically acceptable salt, ester, or solvate thereof with a radionuclide A' or a non-radioactive element A', and the complex having a structure represented by Formula (IV): 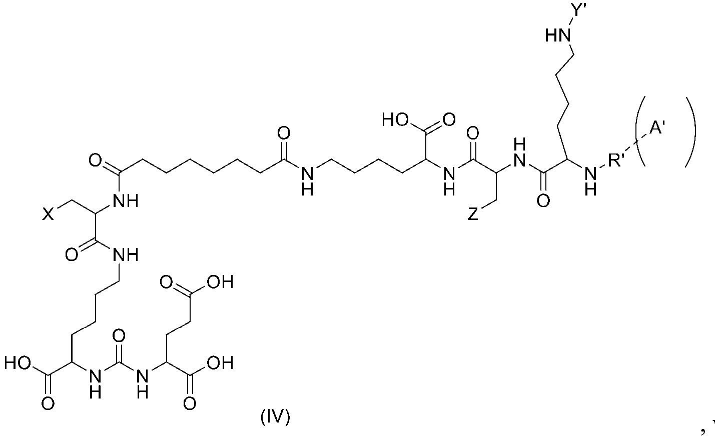 wherein:
R' is a chelating agent; and
Y' is H, an optionally substituted amino acid, or a chelating agent.

6. A compound, being selected from the compound according to any one of claims 1 to 3, or a pharmaceutically acceptable salt, ester, or solvate thereof, wherein the compound has a structure selected from one of: 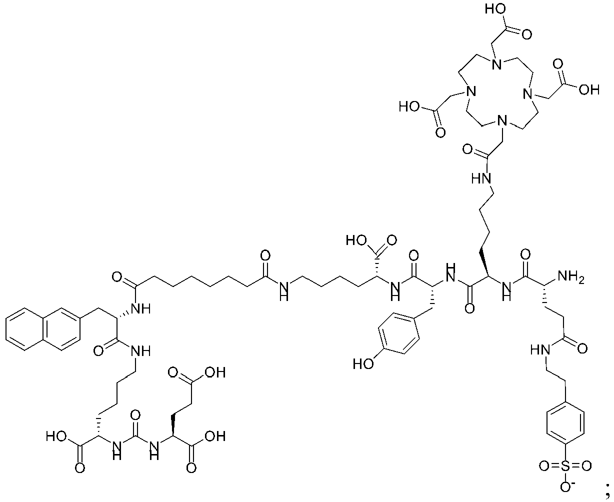 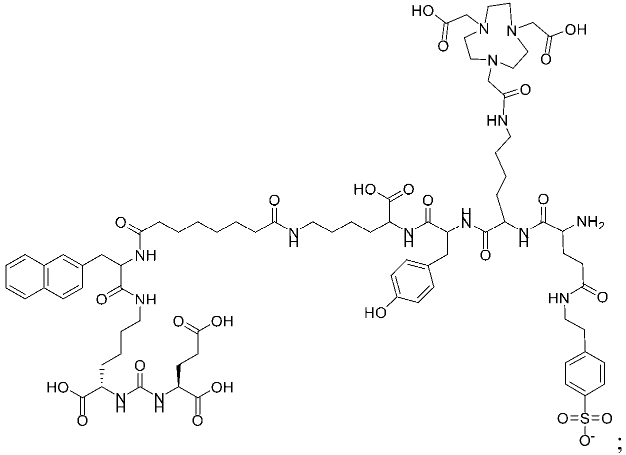 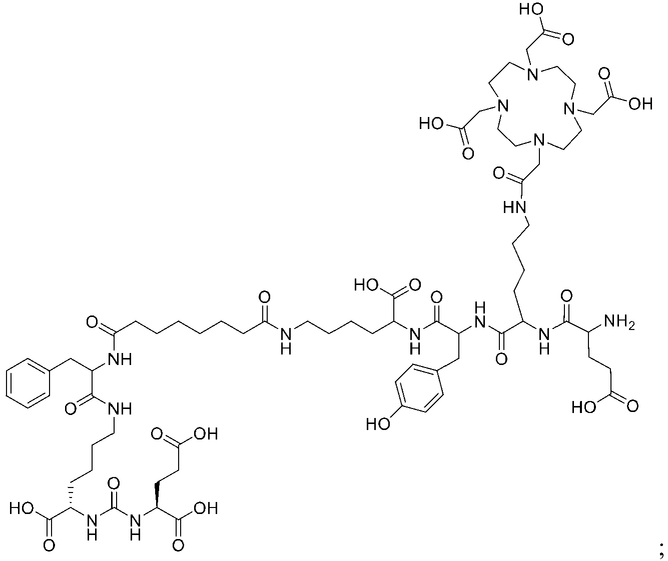 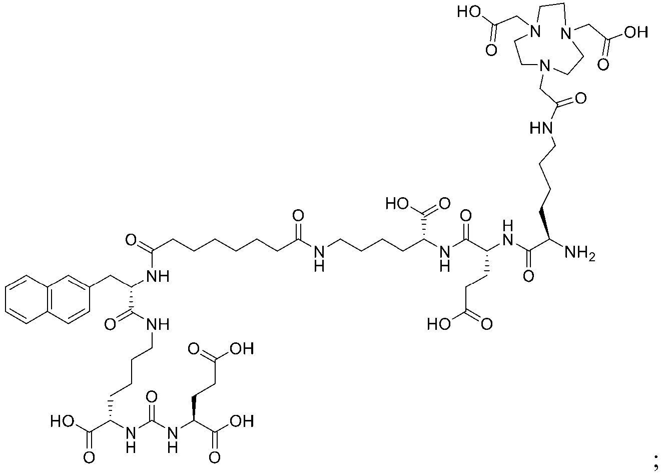 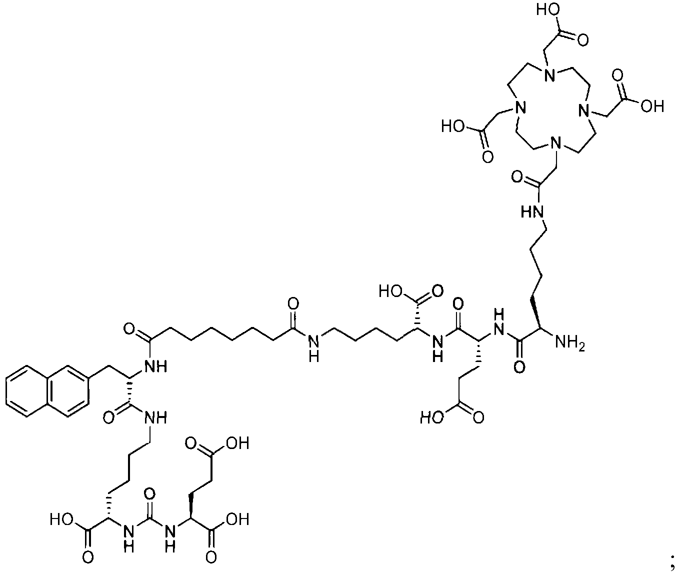 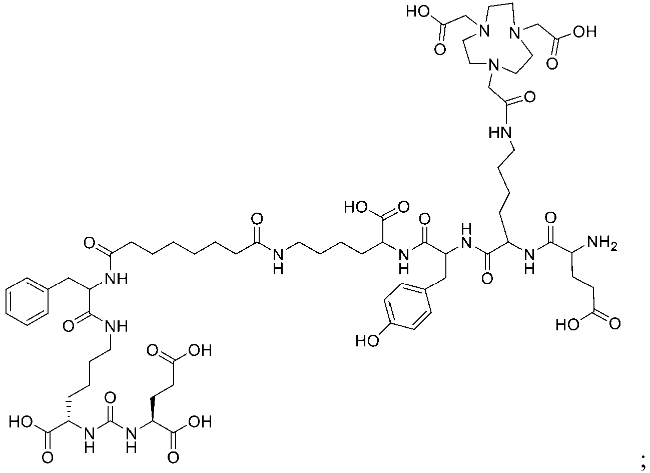 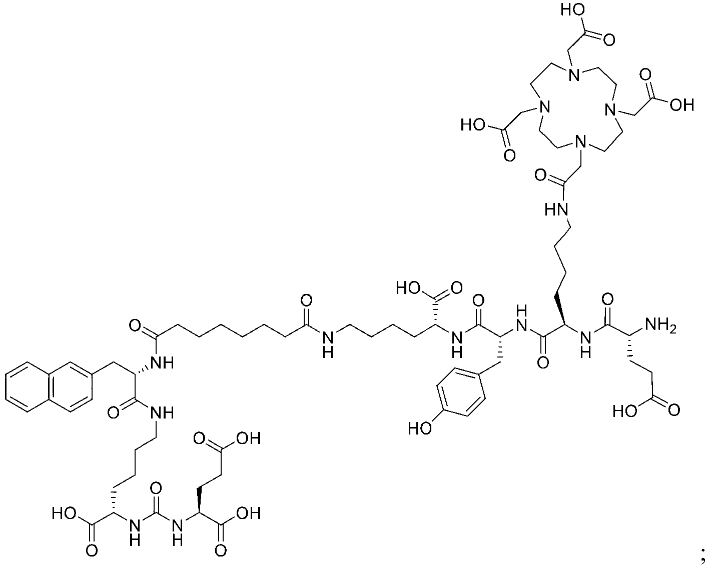 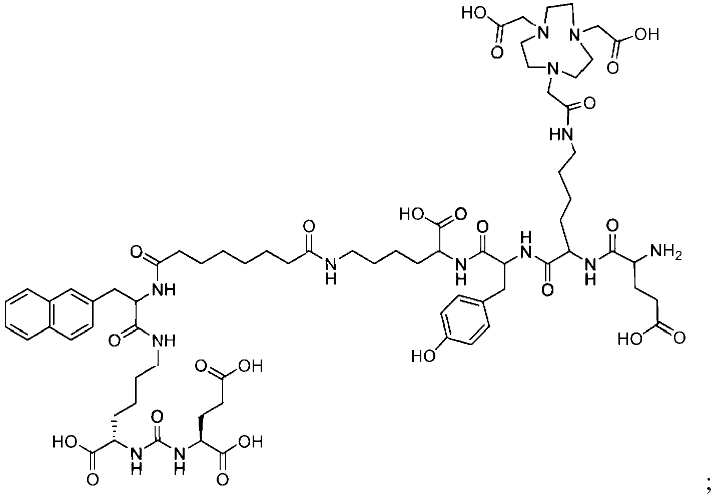 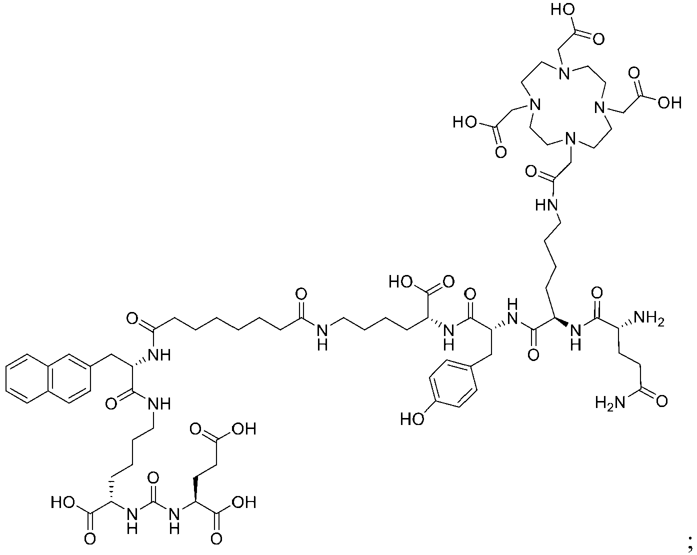 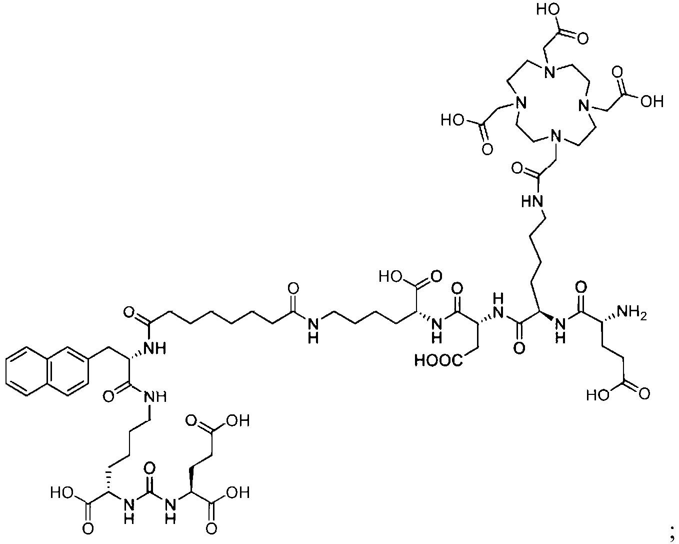 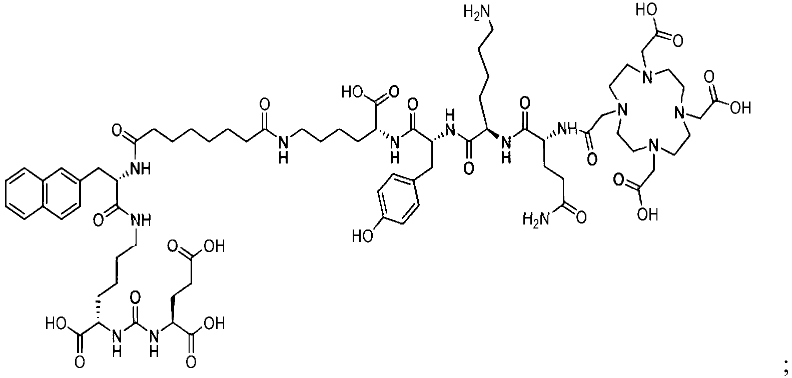 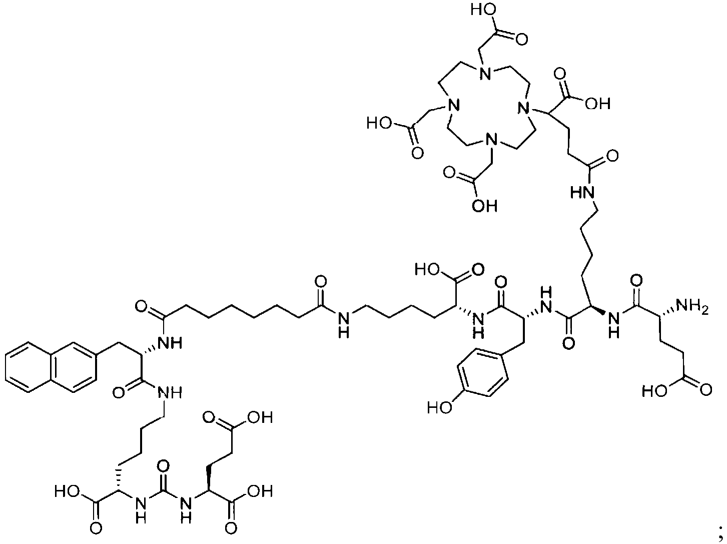 and

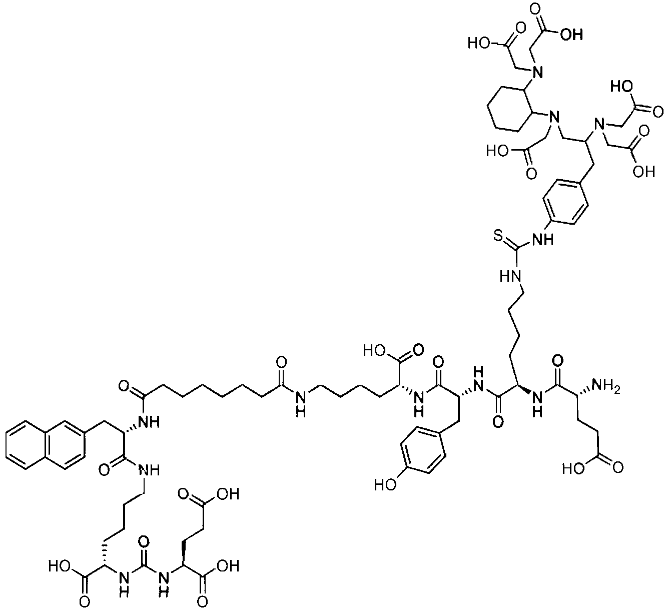

7. A complex, the complex being formed by complexing the compound or the pharmaceutically acceptable salt, ester, or solvate thereof according to any one of claims 1 to 3 and 6 or the complex according to claim 4 or 5 with a radionuclide B' or a non-radioactive element B'.

8. The complex according to claim 4, 5, or 7, wherein the radionuclide A' or the radionuclide B' is respectively and independently selected from at least one of ⁶⁸Ga, ¹⁸F, ^{99m}Tc, ⁸⁹Zr, ¹¹¹In, ⁴⁵Ti, ⁵⁹Fe, ⁶⁴Cu, ^{94m}Tc, ⁶⁷Ga, ^{71/72/74}As, ^{43/44}Sc, ^{82m}Rb, ⁵²Mn, ⁸⁶Y, ⁷⁶Br, ¹⁷⁷Lu, ⁹⁰Y, ¹⁵³Sm, ⁶⁷Cu, ⁸⁹Sr, ¹³⁷Cs, ¹⁶⁶Ho, ¹⁷⁷Yb, ¹⁰⁵Rh, ^{186/188}Re, ⁴⁷Sc, ^{212/213}Bi, ²²⁵Ac, ²¹²Pb, ¹⁴⁹Pm, and ²²⁷Th,
optionally, the non-radioactive element A' or the non-radioactive element B' is each independently selected from at least one of Ga, Fe, and Gd;
optionally, the radionuclide is selected from ¹⁸F, ⁶⁸Ga, or ¹⁷⁷Lu; and
optionally, a complexation of radionuclide ¹⁸F is achieved via radioactive aluminum fluoride ¹⁸F AI.

9. A complex, having a structure selected from one of: 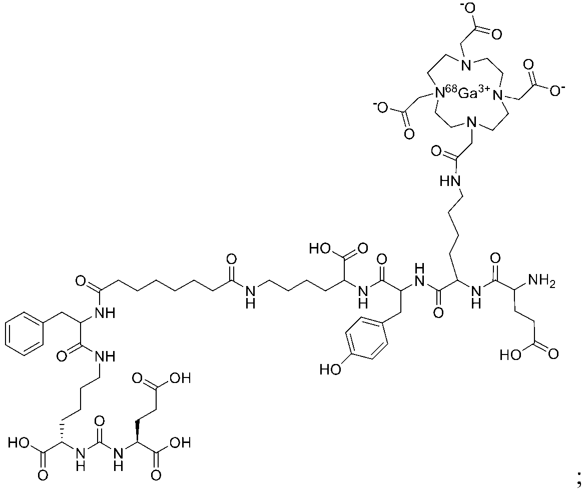 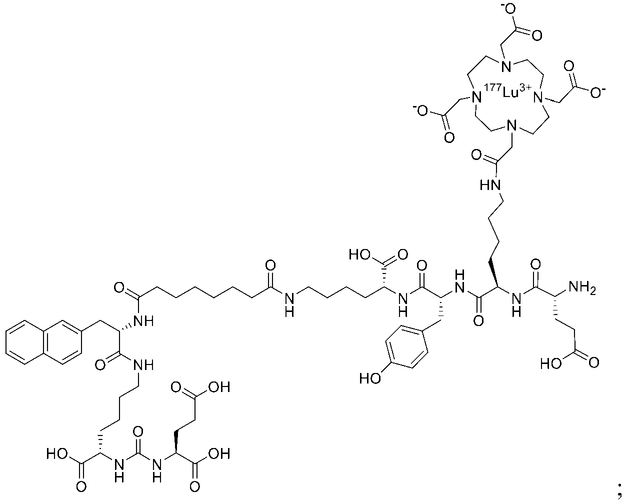 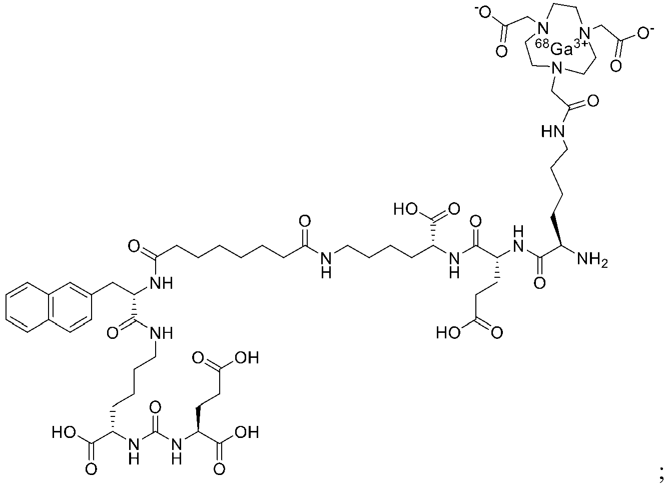 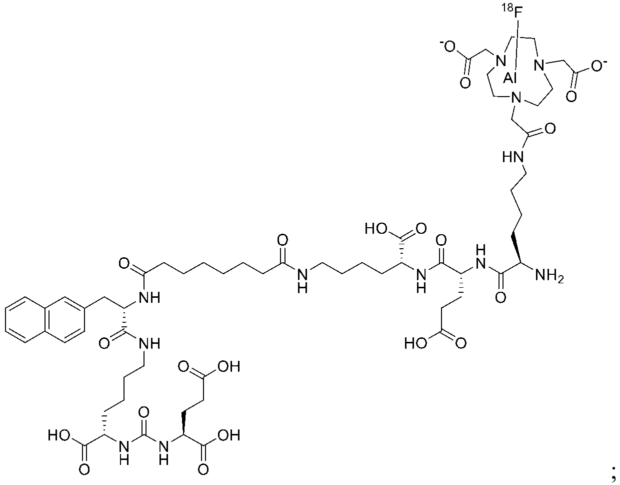 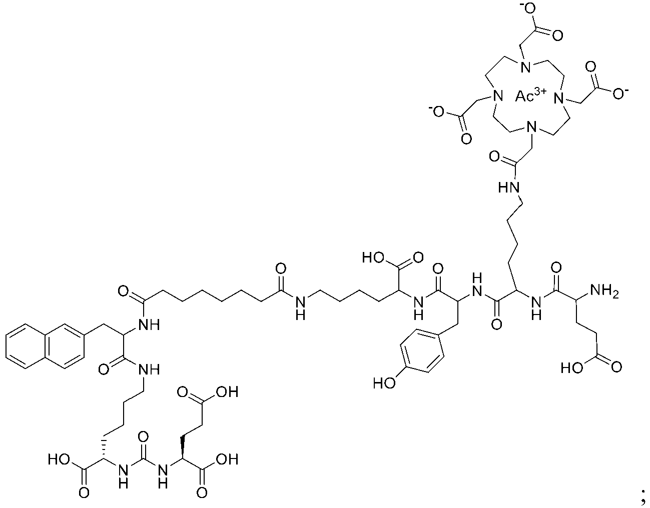 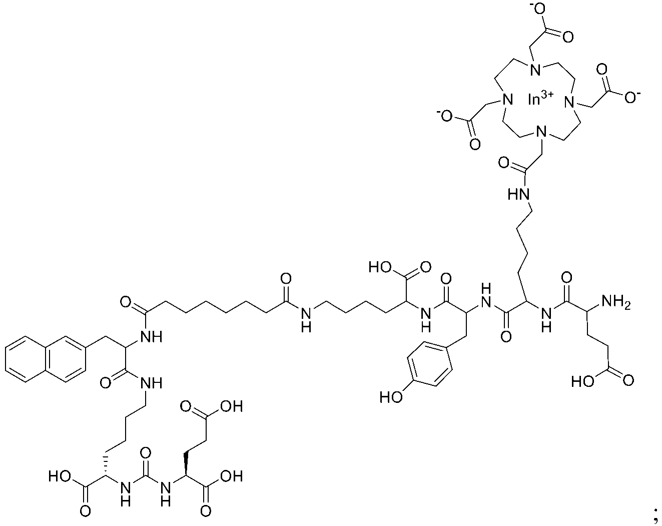 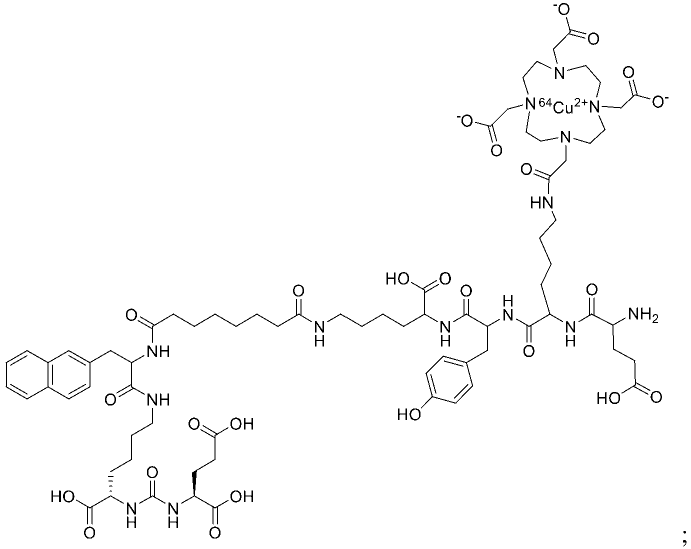 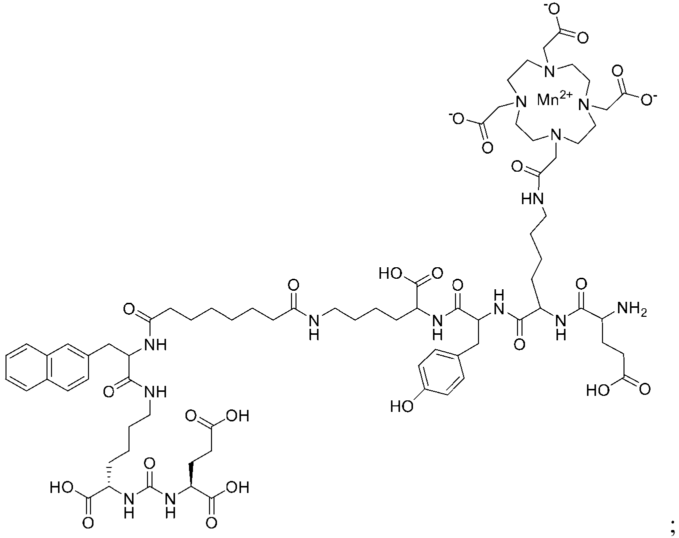 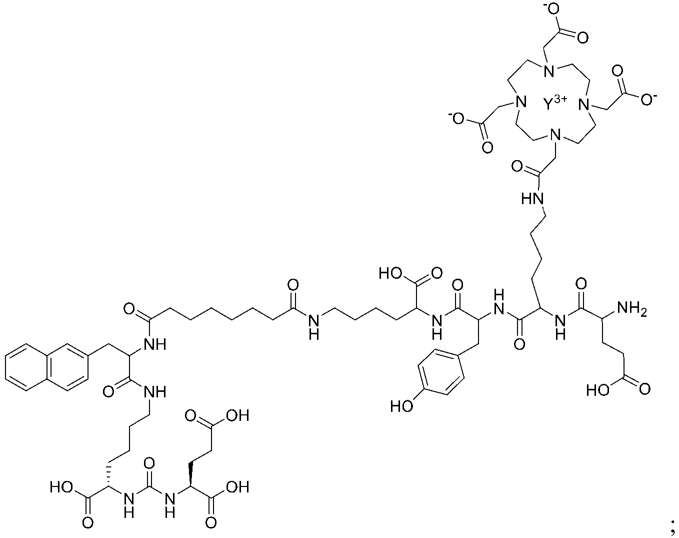 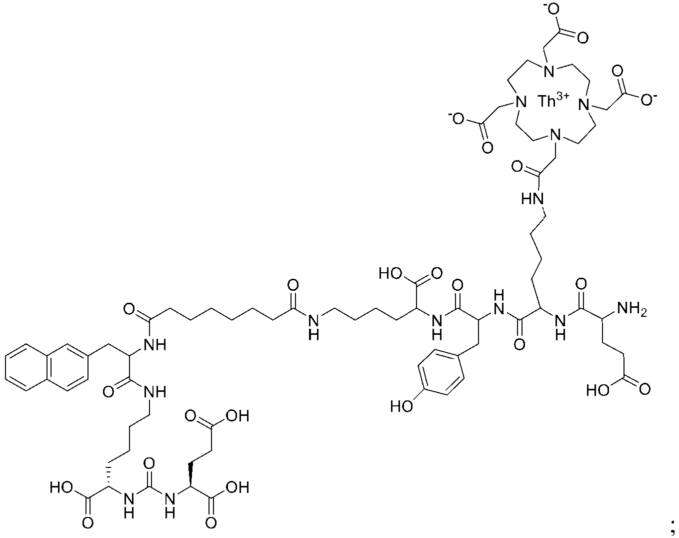 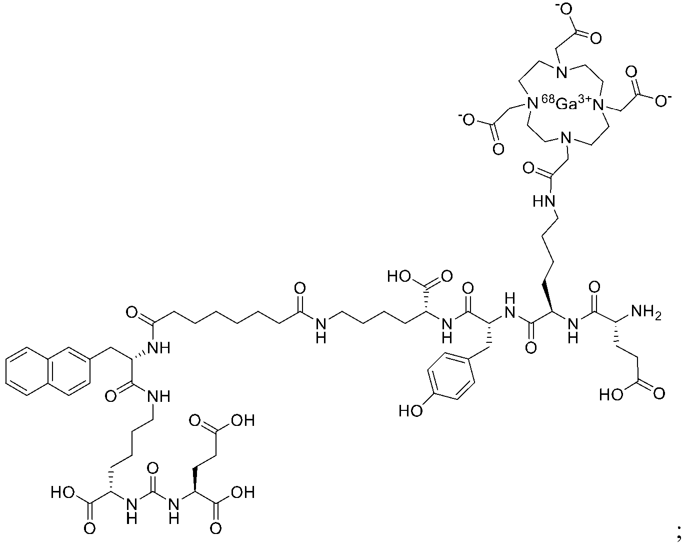 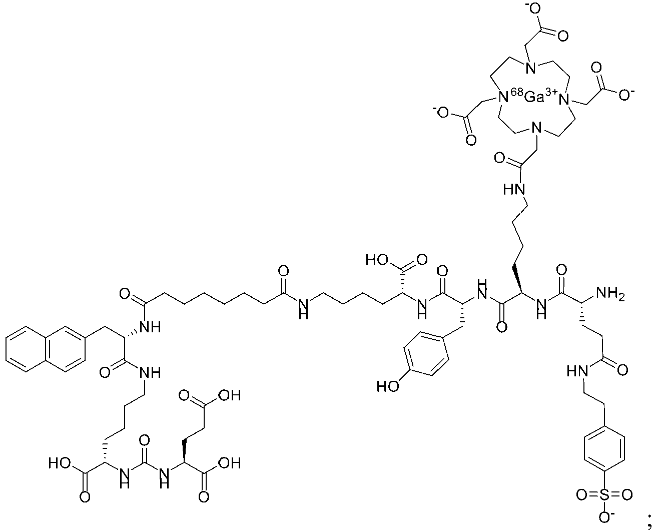 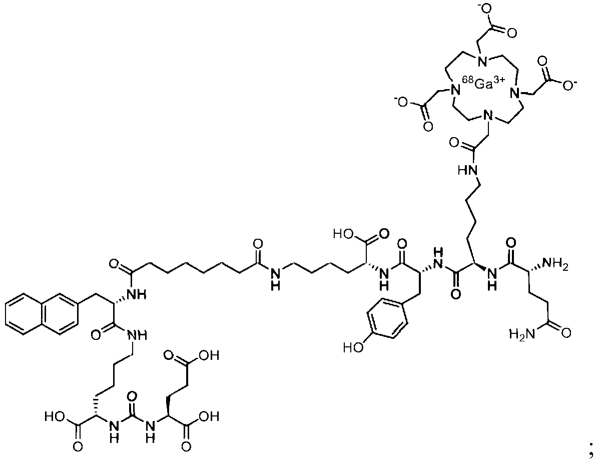 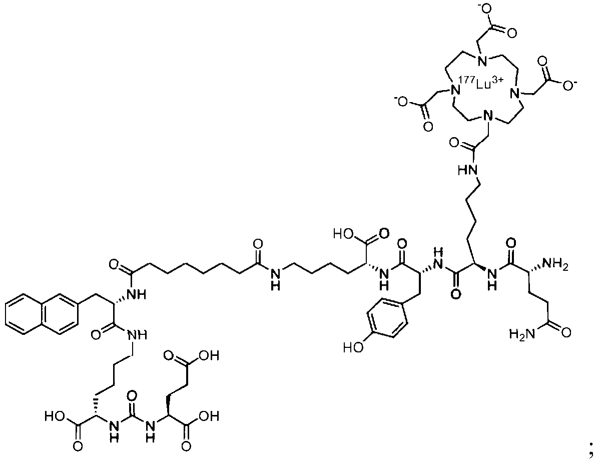 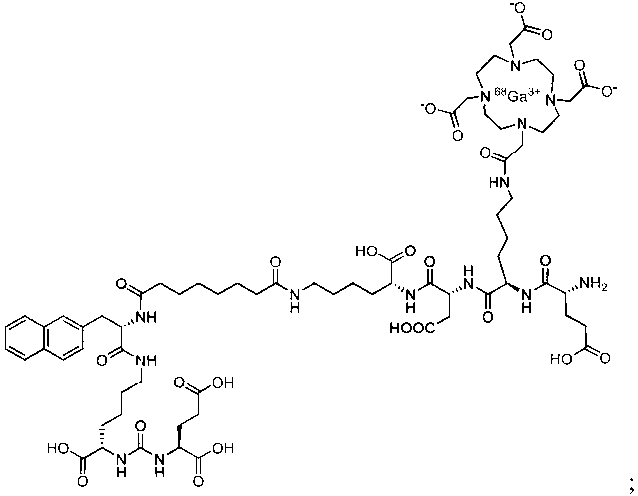 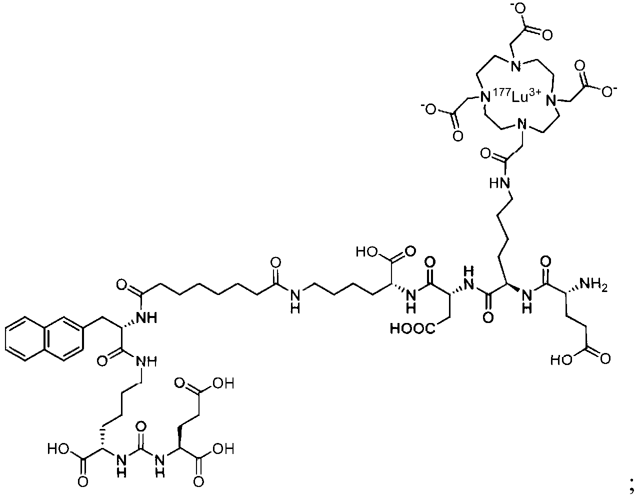 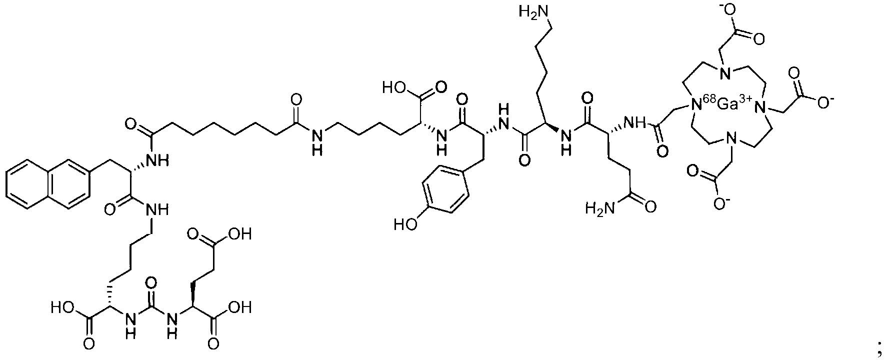 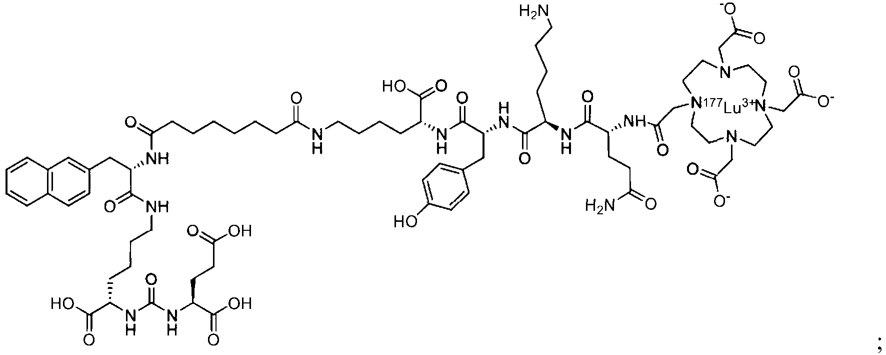 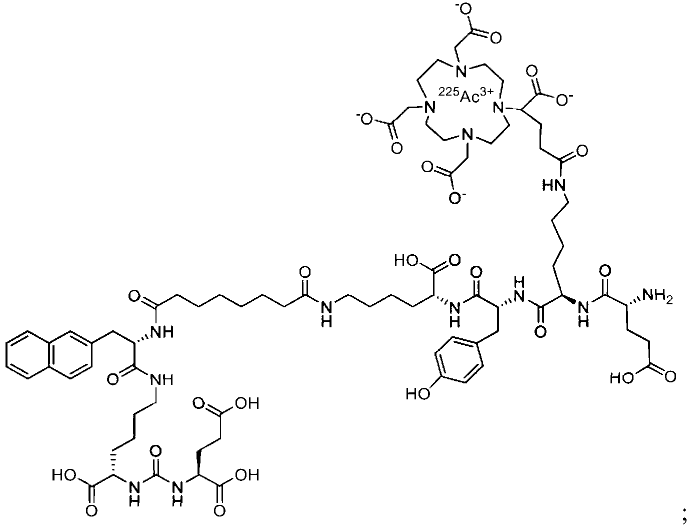 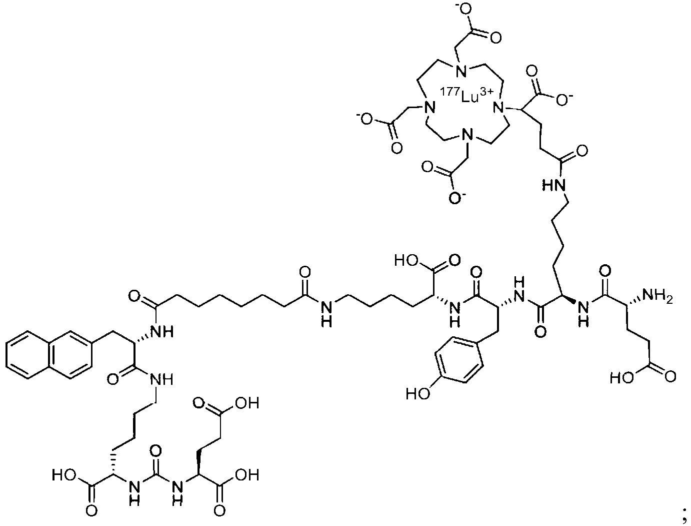 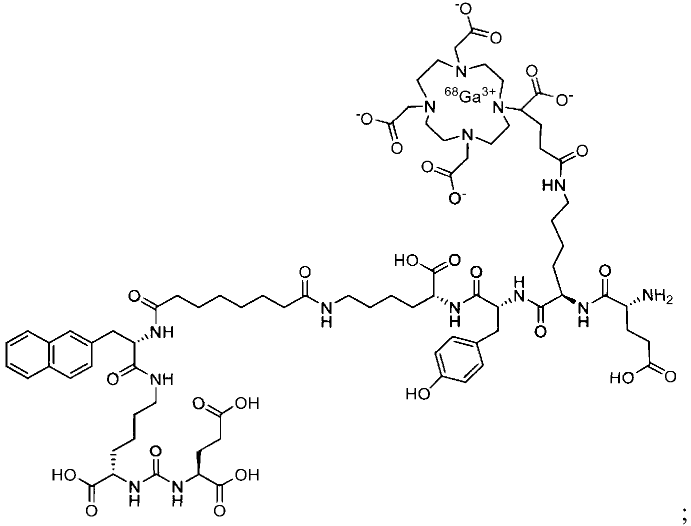 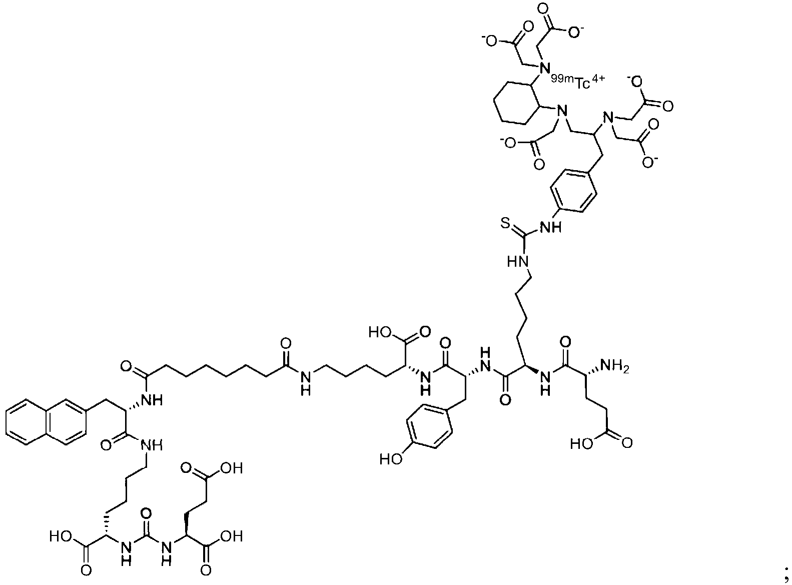 and

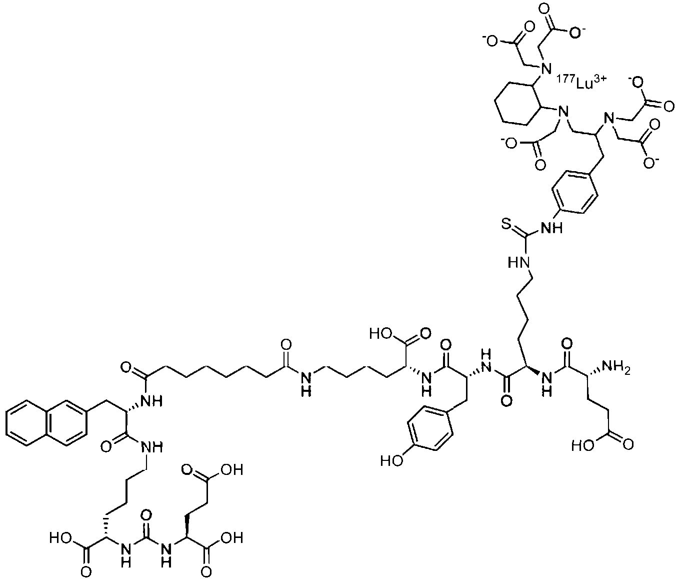

10. A pharmaceutical composition, comprising:
the compound, or the pharmaceutically acceptable salt, ester or solvate thereof according to any one of claims 1 to 3 and 6, or the complex according to any one of claims 4 to 5 and 7 to 9; and
a pharmaceutically acceptable carrier or excipient.

11. Use of the compound, or the pharmaceutically acceptable salt, ester or solvate thereof according to any one of claims 1 to 3 and 6, the complex according to any one of claims 4 to 5 and 7 to 9, or the pharmaceutical composition according to claim 10, in the preparation of one or more reagents and/or medicaments for diagnosing and/or treating a tumor, a cancer, or a cell expressing PSMA.

12. Use of the compound, or the pharmaceutically acceptable salt, ester or solvate thereof according to any one of claims 1 to 3 and 6, the complex according to any one of claims 4 to 5, and 7 to 9, or the pharmaceutical composition according to claim 10, in the diagnosis and/or treatment of a tumor, a cancer, or a cell expressing PSMA.

13. The use according to claim 11 or 12, wherein the diagnosis is implemented by a means selected from optical imaging and/or radionuclide imaging,
optionally, the diagnosis is implemented by a means selected from PET imaging and/or SPECT imaging;
optionally, the treatment is selected from radiotherapy; and
optionally, the cancer is selected from prostate cancer and/or other solid tumors.

14. A method for diagnosis and/or treatment of a tumor, a cancer, or a cell expressing PSMA, the method comprising:
administering to a patient a pharmaceutically acceptable dose of the compound, or the pharmaceutically acceptable salt, ester or solvate thereof according to any one of claims 1 to 3 and 6, or the complex according to any one of claims 4 to 5, and 7 to 9, or the pharmaceutical composition according to claim 10.

15. The method according to claim 14, wherein the diagnosis is implemented by a means selected from optical imaging and/or radionuclide imaging,
optionally, the diagnosis is implemented by a means selected from PET imaging and/or SPECT imaging;
optionally, the treatment is selected from radiotherapy; and
optionally, the cancer is selected from prostate cancer and/or other solid tumors.

16. A kit, comprising:
the compound, or the pharmaceutically acceptable salt, ester or solvate thereof according to any one of claims 1 to 3 and 6, or the complex according to any one of claims 4 to 5, and 7 to 9, and
a pharmaceutically acceptable carrier and excipients,
optionally, the kit further comprising a pharmaceutical excipient, wherein the pharmaceutical excipient comprises sterile water for injection, acetic acid/sodium acetate buffer solution, and sodium ascorbate.
